# FASCICULE DE BREVET EUROPEEN

(11) **EP 1 021 442 B1**
(45) Date de publication et mention de la délivrance du brevet: **30.01.2002**
(21) Numéro de dépôt: 98947615.5
(22) Date de dépôt: 07.10.1998
(51) Int. Cl.: C07D 451/02, A61K 31/36

(54) **DERIVES DE 8-AZABICYCLO 3.2.1] OCTANE-3-METHANAMINE EN TANT QUE LIGANDS DES RECEPTEURS DE DOPAMINE D2 ET D3 ET DE SEROTONINE 5HT1A ET 5HT2**
8-AZABICYCLO(3.2.1)0CTAN-3-METHANAMINDERIVATE ALS LIGANDEN DER DOPAMIN-REZEPTOREN D2 UND D3 UND DER SEROTONIN-REZEPTOREN 5HT1A UND 5HT2
8-AZABICYCLO 3.2.1] OCTANE-3-METHANAMINE DERIVATIVES AS LIGANDS OF D2 AND D3 DOPAMINE AND 5HT1A AND 5HT2 SEROTONIN RECEPTORS

(30) Priorité: 09.10.1997 FR 9712580; 09.10.1997 FR 9712583
(43) Date de publication de la demande: 26.07.2000
(73) Titulaire: SANOFI-SYNTHELABO, 75013 Paris (FR)
(72) Inventeur: MARABOUT, Benoit, F-91380 Chilly Mazarin (FR); SEVRIN, Mireille, F-75014 Paris (FR); GEORGE, Pascal, F-78730 Saint Arnoult en Yvelines (FR); MERLY, Jean-Pierre, F-92330 Sceaux (FR); DE PERETTI, Danièle, F-92160 Antony (FR); ROY, Jocelyne, F-91130 Ris Orangis (FR); MACHNIK, David, F-75014 Paris (FR)
(74) Mandataire: Ludwig, Jacques
(86) Numéro de dépôt international: FR9802137
(87) Numéro de publication internationale: WO9919325

(56) Documents cités:
- EP-A- 0 081 054
- EP-A- 0 447 292
- EP-A- 0 532 398
- EP-A- 0 540 914
- WO-A-94/20466
- FR-A- 2 681 325
- US-A- 4 910 302

## Description

La présente invention a pour objet des composés de formule générale (I) 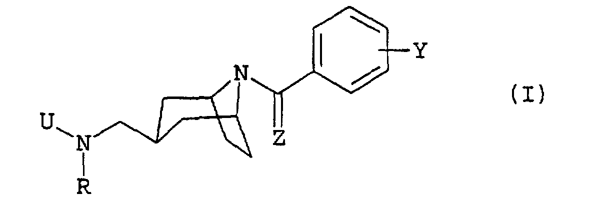 dans laquelle
U représente
A) soit un groupe 2,3-dihydro-1*H*-indén-2-yle de formule générale (A) 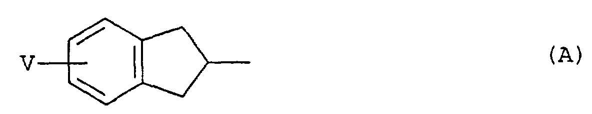
B) soit un groupe hétérocyclique de formule générale (B) 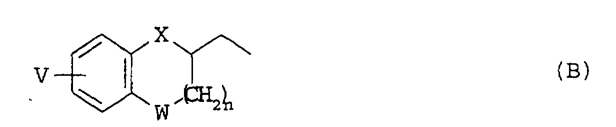 dans lesquelles
   V représente un atome d'hydrogène ou d'halogène, un groupe (C₁-C₃)alkyle ou un ou deux groupes (C₁-C₃)alcoxy,
   W et X représentent chacun, respectivement, soit deux atomes d'oxygène, soit un atome d'oxygène et un groupe CH₂, soit un groupe CH₂ et un atome d'oxygène, soit un atome d'oxygène et un groupe CO,
   n représente le nombre 0 ou 1,
   R représente soit un groupe propyle lorsque U représente un groupe 2,3-dihydro-1H-indén-2-yle de formule générale (A), soit un atome d'hydrogène ou un groupe (C₁-C₃)alkyle lorsque U représente un groupe hétérocyclique de formule générale (B),
   Y représente un ou plusieurs atomes ou groupes choisis parmi les suivants : hydrogène, halogène, (C₁-C₃)alkyle et (C₁-C₃)alcoxy,
   Z représente deux atomes d'hydrogène ou un atome d'oxygène.

Les composés de l'invention peuvent exister sous deux formes d'isomères géométriques, à savoir la forme isomère α, ou endo, dans laquelle le groupe CH₂ en position 3 du cycle tropane (azabicyclooctane) est en position axiale, et la forme isomère β, ou exo, dans laquelle ledit groupe CH₂ est en position équatoriale, dans la conformation dite "chaise" du motif pipéridine du cycle tropane.

Les composés de l'invention peuvent aussi exister à l'état de bases ou de sels d'addition à des acides.

Lorsque U représente un groupe 2,3-dihydro-1*H*-indén-2-yle de formule générale (A), les composés de l'invention répondent à la formule générale (IA)

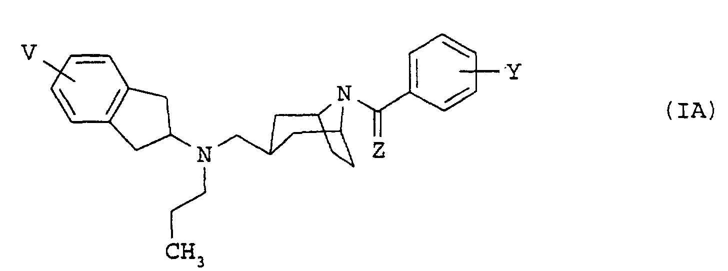

Ils peuvent être préparés selon un procédé illustré par le schéma 1A qui suit.

On fait réagir le 8-(phénylméthyl)-8-azabicyclo[3.2.1]octane-3-carboxylate d'éthyle de formule (IIA) avec un amidure de diméthylaluminium, préalablement préparé à partir de triméthylaluminium et d'un dérivé de 2,3-dihydro-1*H*-indène-2-amine de formule générale (IIIA), dans laquelle V est tel que défini ci-dessus, dans un solvant inerte, par exemple le toluène, à une température de 0 à 100°C ; on obtient un composé de formule générale (IVA) que l'on réduit par action d'un hydrure alcalin mixte tel que 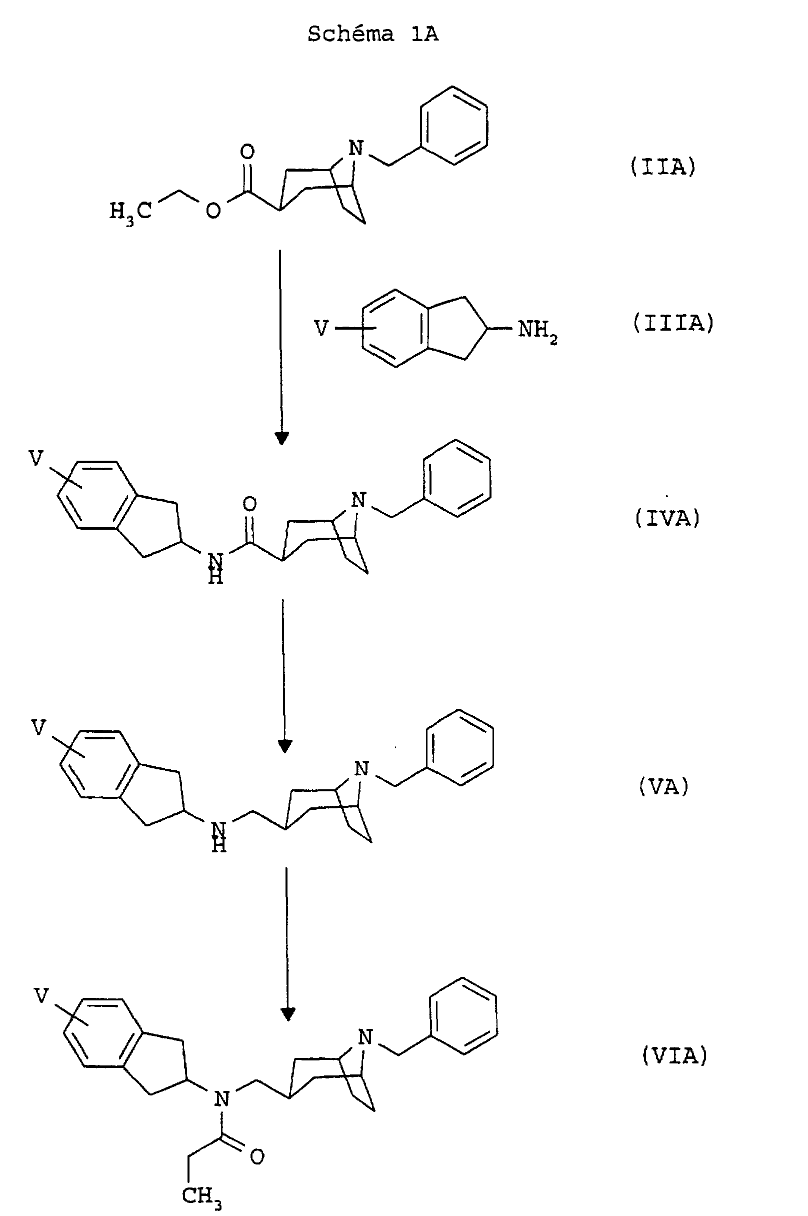 l'hydrure d'aluminium et de lithium, dans un solvant éthéré, par exemple le tétrahydrofurane, à une température de 0 à 60°C, pour obtenir un composé de formule générale (VA). On soumet ce dernier à une acylation au moyen de chlorure de propanoyle, dans un solvant chloré, par exemple le dichlorométhane, en présence d'une base telle que la triéthylamine, à une température de 0 à 40°C, pour obtenir 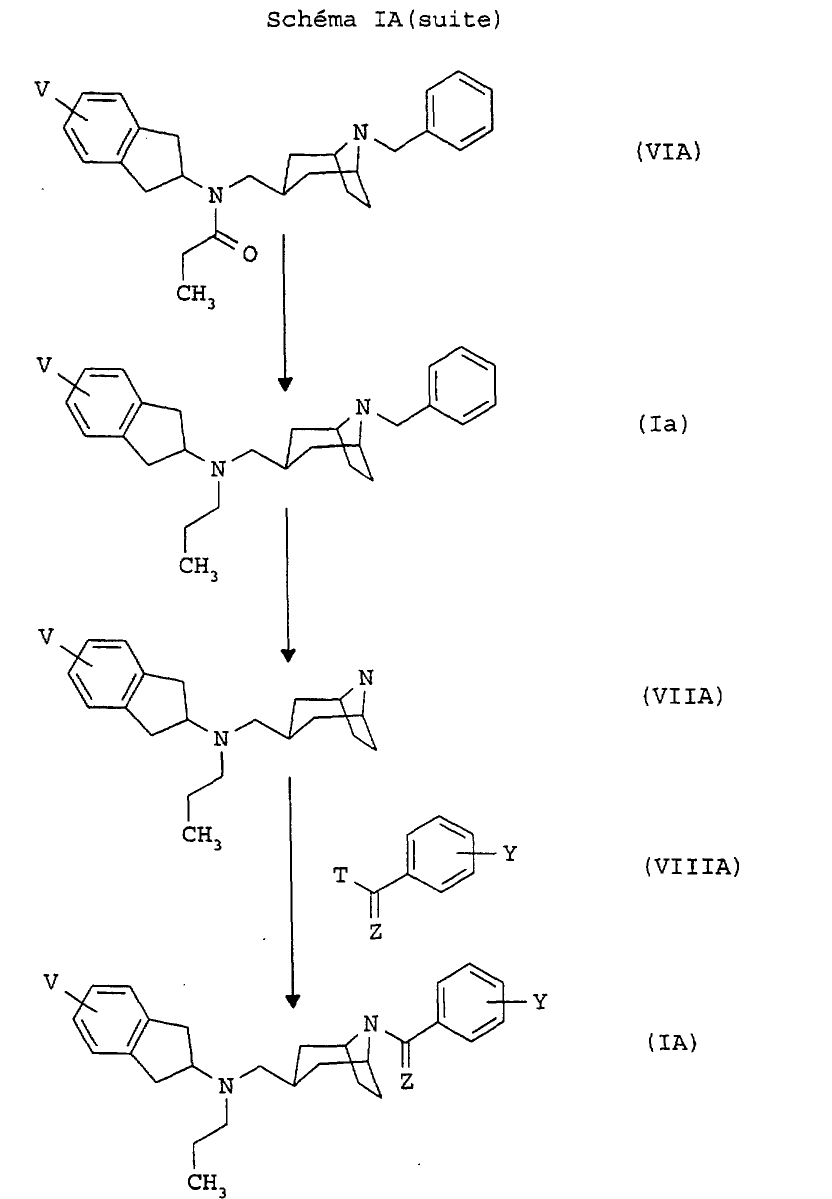 un amide de formule générale (VIA), que l'on réduit par action d'un hydrure alcalin mixte tel que l'hydrure d'aluminium et de lithium, dans un solvant éthéré, par exemple le tétrahydrofurane, à une température de 0 à 60°C, pour obtenir un composé de formule générale (Ia), qui correspond à la formule générale (IA) lorsque Y représente un atome d'hydrogène et Z représente deux atomes d'hydrogène. Pour préparer un autre composé de formule générale (IA) on effectue ensuite une débenzylation, par exemple par hydrogénation catalytique, pour obtenir l'amine de formule générale (VIIA), et finalement on fait réagir cette dernière soit avec un chlorure d'acide de formule générale (VIIIA) dans laquelle Y est tel que défini ci-dessus, Z représente un atome d'oxygène et T représente un atome de chlore, dans un solvant chloré, par exemple le dichlorométhane, en présence d'une base telle que la triéthylamine, à une température de 20 à 40°C, soit avec un dérivé halogéné de formule générale (VIIIA) dans laquelle Y est tel que défini ci-dessus, Z représente deux atomes d'hydrogène et T représente un atome d'halogène, dans un solvant aprotique, par exemple le *N,N*-diméthylformamide, en présence d'une base telle que le carbonate de potassium, à une température de 20 à 100°C.

La 2,3-dihydro-1*H*-indène-2-amine de formule générale (IIIA) dans laquelle X représente l'hydrogène est disponible dans le commerce ; les dérivés substitués de 2,3-dihydro-1*H*-indène-2-amine de formule générale (IIIA) peuvent être préparés par des méthodes analogues à celles décrites dans Can. *J. Chem.* (1974) **52** 381-389.

Le 8-(phénylméthyl)-8-azabicyclo[3.2.1]octane-3-carboxylate d'éthyle de formule (IIA) peut être préparé par une méthode analogue à celle décrite dans *J. Med. Chem.* (1994) **37** 2831.

Lorsque U représente un groupe hétérocyclique de formule générale (B), les composés de l'invention répondent à la formule générale (IB)

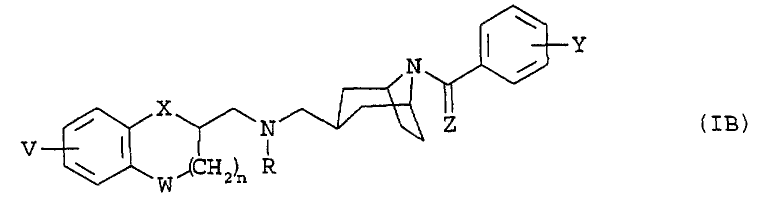

Ils peuvent être préparés selon des procédés illustrés par les schémas 1B à 3B qui suivent.

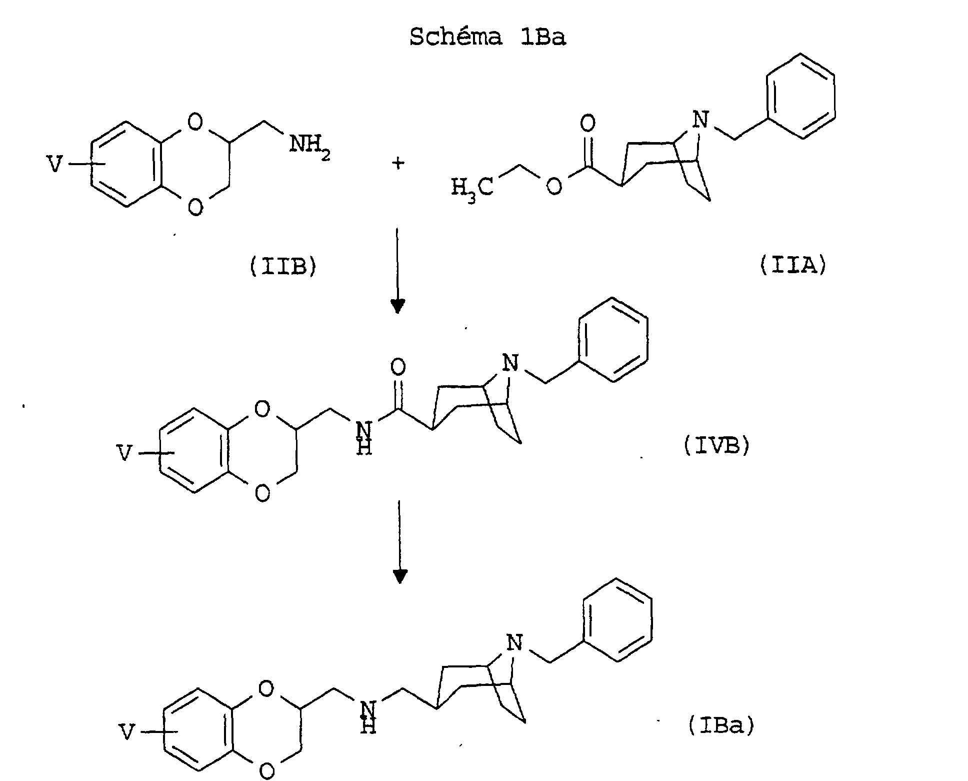

Selon les schémas 1Ba, 1Bb et 1Bc, on prépare les composés de formule générale (IB) dans laquelle W et X représentent chacun un atome d'oxygène et n représente le nombre 1 en faisant réagir le 8-(phénylméthyl)-8-azabicyclo[3.2.1]-octane-3-carboxylate d'éthyle de formule (IIA) avec un amidure de diméthylaluminium, préalablement préparé à partir de triméthylaluminium et d'un dérivé de 2,3-dihydro-1,4-benzodioxane-2-méthanamine de formule générale (IIB), dans laquelle V est tel que défini ci-dessus, dans un solvant inerte tel que le toluène, à une température de 0 à 100°C, pour obtenir un composé de formule générale (IVB), qu'on réduit par action d'un hydrure alcalin mixte, par exemple l'hydrure d'aluminium et de lithium, dans un solvant éthéré tel que le tétrahydrofurane, à une température de 0 à 60°C.
On obtient un composé de formule générale (IBa) qui correspond à la formule générale (IB) où R et Y représentent chacun un atome d'hydrogène et Z représente deux atomes d'hydrogène.

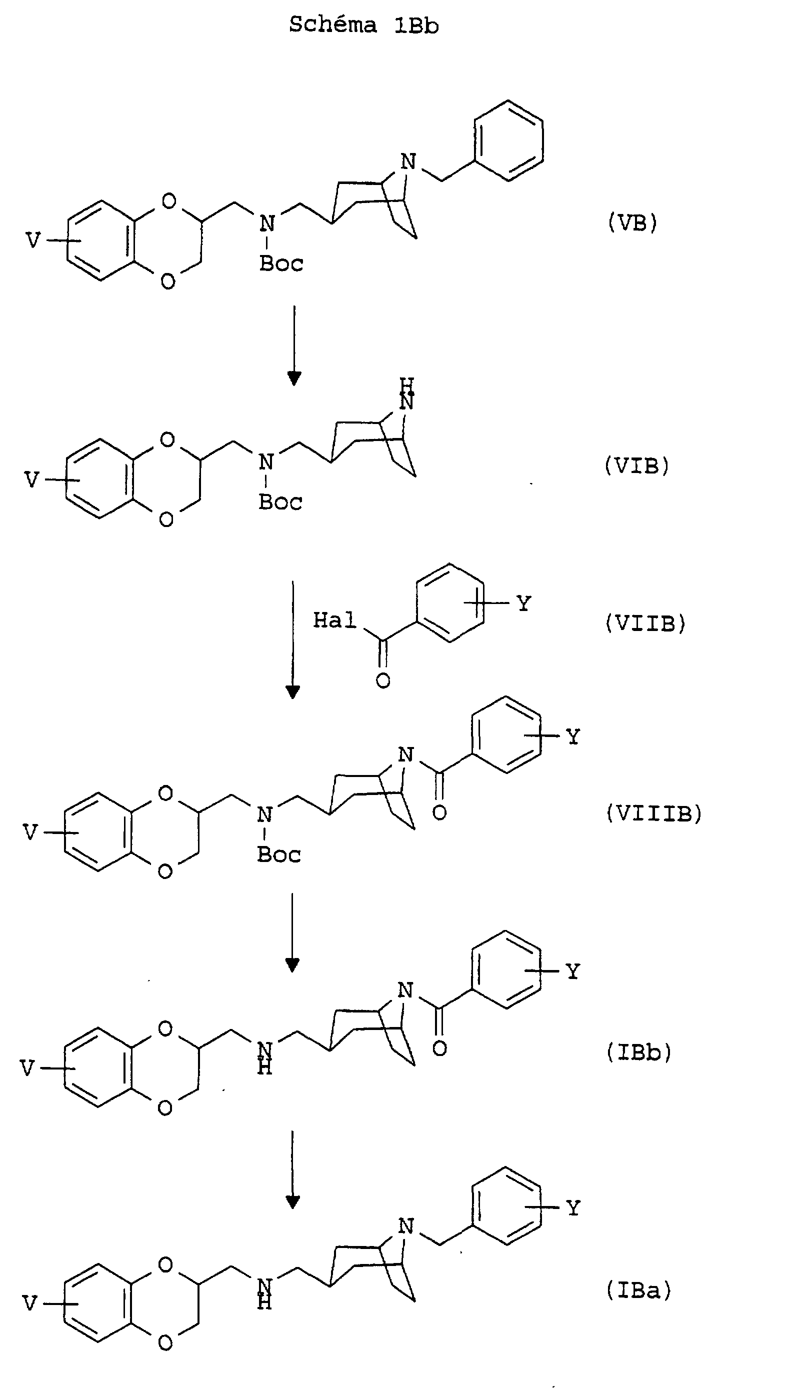

Selon que l'on désire un composé final dans la formule générale duquel R représente un atome d'hydrogène ou un groupe alkyle, on traite ensuite le composé de formule générale (IBa) par l'un des procédés illustrés par les schémas 1Bb ou 1Bc.

Selon le schéma 1Bb on commence par protéger la fonction amine secondaire du composé de formule générale (IBa) par action du dicarbonate de bis(1,1-diméthyléthyle), dans un solvant chloré tel que le dichlorométhane, pour obtenir un composé de formule générale (VB), dans laquelle Boc représente un groupe 1,1-diméthyléthoxycarbonyle. On débenzyle ce composé par hydrogénation catalytique, puis on fait réagir le composé ainsi obtenu, de formule générale (VIB), avec avec un chlorure d'acide de formule générale (VIIB) dans laquelle Y est tel que défini ci-dessus et Hal représente un atome de chlore, dans un solvant chloré, par exemple le dichlorométhane, en présence d'une base telle que la triéthylamine, à une température de 20 à 40°C.
On obtient un composé de formule générale (VIIIB) dont on déprotège la fonction amine secondaire au moyen d'acide trifluoroacétique, pour obtenir un composé de formule générale (IBb).
Finalement, et si on le désire, on réduit ce composé par action d'un hydrure alcalin mixte, par exemple l'hydrure d'aluminium et de lithium, dans un solvant éthéré tel que le tétrahydrofurane, pour obtenir un composé de formule générale (IBa).

Selon le schéma 1Bc on soumet un composé de formule générale (IBa) à une acylation au moyen d'anhydride mixte acétique-formique ou d'un chlorure d'acide en C₂-C₃, dans un solvant chloré tel que le dichlorométhane, à une température de 0 à 40°C, pour obtenir un composé de formule générale (IXB), dans laquelle R' représente un atome d'hydrogène ou un groupe méthyle ou éthyle, puis on réduit ce dernier au moyen d'un hydrure alcalin mixte, par exemple l'hydrure d'aluminium et de lithium, dans un solvant éthéré tel que le tétrahydrofurane, pour obtenir un composé de 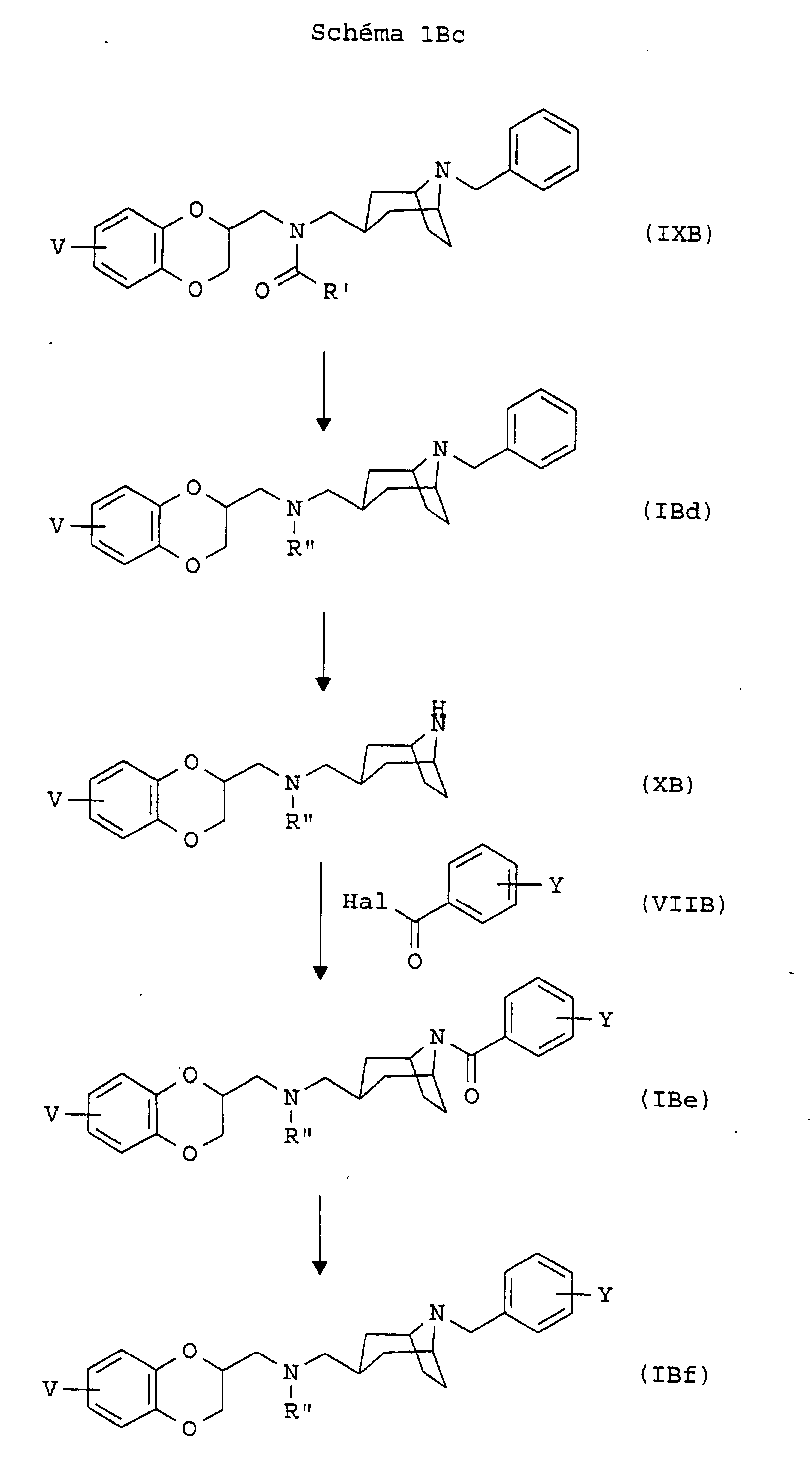 formule générale (IBd), dans laquelle R" représente un groupe (C₁-C₃) alkyle.
On traite ensuite ce composé comme indiqué à propos du composé de formule générale (VB), à l'exception, bien entendu, de l'étape de déprotection de l'amine.

Selon le schéma 2B, on prépare les composés de formule générale (IB) dans laquelle X représente un atome d'oxygène, W représente un atome d'oxygène ou un groupe CH₂, R représente un atome d'hydrogène et n représente le nombre 1 en faisant réagir un composé de formule générale (XIB), dans laquelle V est tel que défini ci-dessus et G représente un groupe partant tel qu'un atome d'halogène ou un groupe méthanesulfonyloxy ou 4-méthylbenzènesulfonyloxy, avec une 8-azabicyclo[3.2.1]octane-3-amine de formule générale (XIIB), dans laquelle Y, Z et R sont tels que définis ci-dessus, dans un solvant tel que l'acétonitrile, en présence d'une base telle que le carbonate de potassium.

Selon le schéma 3B, on prépare les composés de formule générale (IB) dans laquelle X représente un groupe CO, W représente un atome d'oxygène, R représente un atome d'hydrogène et n représente le nombre 1, en faisant réagir un composé de formule générale (XIIIB), dans laquelle V est tel que défini ci-dessus, avec le paraformaldéhyde et une 8-azabicyclo[3.2.1]octane-3-amine de formule générale (XIIB), dans laquelle Y, Z et R sont tels que définis ci-dessus, dans un solvant tel que le propan-2-ol, en présence d'une quantité catalytique d'acide chlorhydrique.

Les composés de départ à utiliser dans les procédés illustrés par les schémas 1B à 3B sont disponibles dans le commerce ou bien peuvent être préparés selon des méthodes identiques ou analogues à celles décrites dans la littérature, notamment dans les demandes de brevets EP-0 193 400 et EP-0 013 138 et dans *J. Med. Chem.* (1983) **26** 823, *J. Med. Chem.* (1989) **32** 1402, *J. Med. Chem.* (1994) **37** 2831.

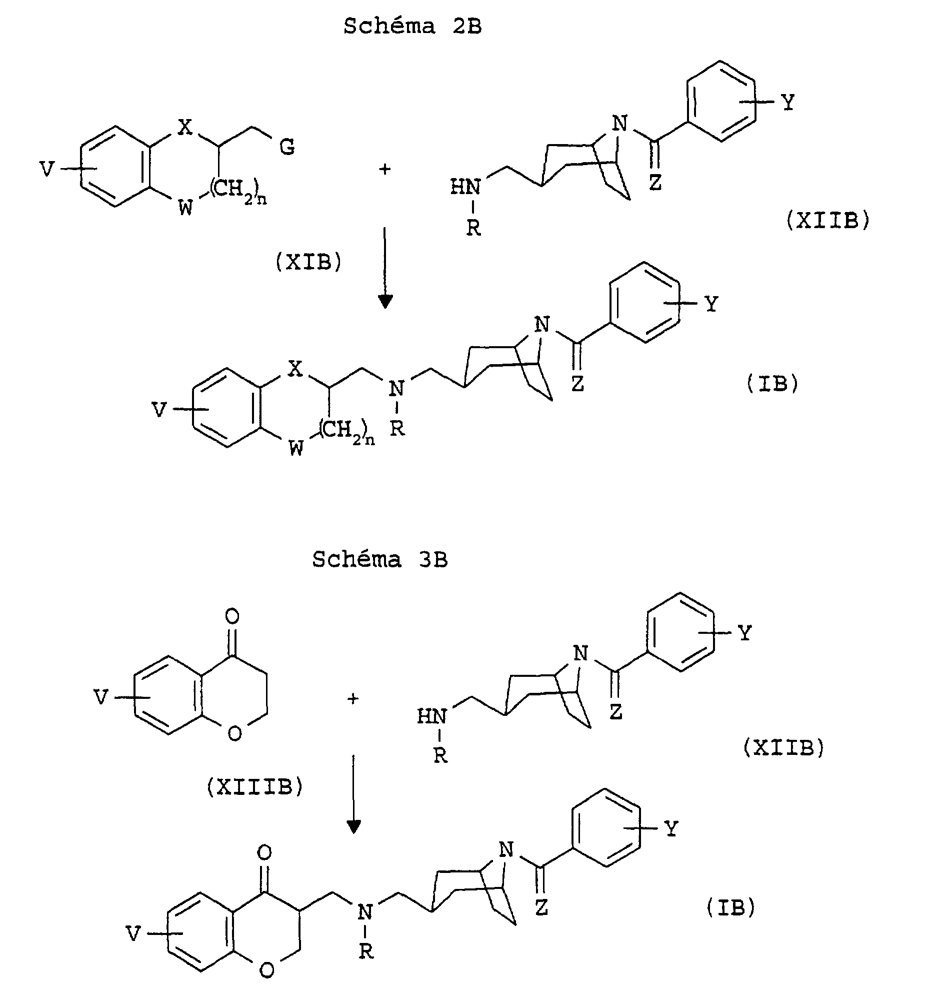

Les exemples qui vont suivre illustrent la préparation de quelques composés de l'invention. Les microanalyses élémentaires, et les spectres I.R. et R.M.N. confirment les structures des composés obtenus.
Les numéros indiqués entre parenthèses dans les titres des exemples correspondent à ceux de la 1ère colonne des tableaux A et B donnés plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1A (Composé N°4A).

(*E*)-But-2-ènedioate de *exo-N*-(2,3-dihydro-1*H*-indén-2-yl)-8-(phénylméthyl)-*N*-propyl-8-azabicyclo[3.2.1]octane-3-méthanamine (5:2).

### 1A.1. exo-N-(2,3-dihydro-1H-indén-2-yl)-8-(phénylméthyl)-8-azabicyclo[3.2.1]octane-3-carboxamide.

Dans un ballon tricol de 250 ml, sous atmosphère d'argon, on introduit 20 ml de toluène, on ajoute lentement 1,48 g (20,4 mmoles) de triméthylaluminium en solution 2 M dans l'heptane, on refroidit le mélange à 0°C avec un bain de glace, eau et sel, on ajoute, goutte à goutte, 3,64 g (27,4 mmoles) de 2,3-dihydro-1*H*-indèn-2-amine, on chauffe le mélange à 50°C pendant quelques minutes puis, à cette température, on ajoute 3,6 g (13,2 mmoles) de 8-(phénylméthyl)-8-azabicyclo[3.2.1]octane-3-carboxylate d'éthyle, et on chauffe le mélange au reflux pendant 4 h.
On le refroidit à 0°C, on l'hydrolyse en ajoutant 24 ml d'eau, on le filtre sur terre d'infusoires, on sèche le filtrat sur sulfate de sodium, on le filtre et on évapore les solvants sous pression réduite.
On purifie le résidu d'évaporation par chromatographie sur colonne de gel de silice en éluant avec un mélange 97/3 à 93/7 de dichlorométhane et de méthanol.
On obtient 4,56 g de solide.
Point de fusion : 129°C.

### 1A.2. (E)-But-2-ènedioate de exo-N-(2,3-dihydro-lH-indén-2-yl)-8-(phénylméthyl)-8-azabicyclo[3.2.1]octane-3-méthanamine (2:1).

Dans un ballon tricol de 250 ml, sous atmosphère d'argon, on introduit 0,56 g (14,7 mmoles) d'hydrure d'aluminium et de lithium en suspension dans 30 ml de tétrahydrofurane, on refroidit le mélange à 0°C, on ajoute 2,65 g (7,35 mmoles) de *exo-N*-(2,3-dihydro-1*H*-indén-2-yl)-8-(phénylméthyl)-8-azabicyclo[3.2.1]octane-3-carboxamide en solution dans 50 ml de tétrahydrofurane et on chauffe le mélange au reflux pendant 12 h.
On refroidit le mélange à 0°C, on hydrolyse l'excès d'hydrure avec de la soude aqueuse 1 N, on élimine l'insoluble par filtration, on sèche le filtrat sur sulfate de sodium, on le filtre et on le concentre sous pression réduite.
On obtient 2,55 g de produit huileux dont on prépare le sel par addition de 1,7 g (14,7 mmoles) d'acide fumarique en solution dans 200 ml d'éthanol à une solution de 2,55 g de la base dans 50 ml d'éthanol, on évapore le solvant sous pression réduite et on recristallise le résidu dans un mélange 4/1 de méthanol et d'éthanol.
On obtient 2,9 g de fumarate.
Point de fusion : 216,5-219°C.

### 1A.3. Chlorhydrate de exo-N- (2,3-dihydro-1H-indén-2-yl)-N-[[8-(phénylméthyl)-8-azabicyclo[3.2.1]oct-3-yl]méthyl]propanamide.

Dans un ballon tricol de 250 ml, sous atmosphère d'argon, on introduit 0,95 g (2,74 mmoles) de *exo*-*N*-(2,3-dihydro-1*H*-indén-2-yl)-8-(phénylméthyl)-8-azabicyclo[3.2.1]octane-3-méthanamine en solution dans 15 ml de dichlorométhane, on ajoute 0,31 g (3,02 mmoles) de triéthylamine et enfin, goutte à goutte, 0,27 g (2,88 mmoles) de chlorure de propanoyle en solution dans 5 ml de dichlorométhane et on laisse le mélange sous agitation à température ambiante pendant 5 h.
On lave le mélange avec trois fois 50 ml d'eau, on sèche la phase organique sur sulfate de sodium, on la fitre, on la concentre sous pression réduite et on purifie le résidu par chromatographie sur colonne de gel de silice en éluant avec un mélange 98/2 à 92/8 de dichlorométhane et de méthanol. On obtient 0,9 g de base.
On prépare le chlorhydrate par addition de 25 ml d'acide chlorhydrique, en solution 0,1 N dans le propan-2-ol, à une solution de 0,9 g (22,4 mmoles) de base dans 10 ml d'acétate d'éthyle. On évapore les solvants sous pression réduite et on recristallise le résidu dans un mélange 9/1 d'acétate d'éthyle et de propan-2-ol.
On obtient 0,65 g de solide blanc.
Point de fusion : 208-210°C.

### 1A.4. (E)-But-2-ènedioate de exo-N- (2,3-dihydro-1H-indén-2-yl) -8- (phénylméthyl) -N-propyl-8-azabicyclo[3.2.1]. octane-3-méthanamine (5:2).

Dans un ballon tricol de 250 ml, sous atmosphère d'argon, on introduit 0,34 g (8,94 mmoles) d'hydrure d'aluminium et de lithium en suspension dans 20 ml de tétrahydrofurane, on refroidit le mélange à 0°C, on ajoute 1,8 g (4,47 mmoles) de *exo-N-* (2,3-dihydro*-*1*H*-indén-2-yl)*-N-* [[8-(phénylméthyl)-8-azabicyclo[3.2.1] oct-3-yl]méthyl]propanamide en solution dans 40 ml de tétrahydrofurane et on chauffe le mélange au reflux pendant 6 h.
On le refroidit à 0°C, on hydrolyse l'excès d'hydrure avec de la soude aqueuse 1 N, on élimine l'insoluble par filtration, on sèche le filtrat sur sulfate de sodium, on le filtre et on le concentre sous pression réduite.
On obtient 1,6 g de produit huileux.
On prépare le difumarate par addition de 0,96 g (8,24 mmoles) d'acide fumarique, en solution dans 250 ml d'éthanol, à une solution de 1,6 g (4,12 mmoles) de base dans 100 ml d'éthanol. On évapore le solvant sous pression réduite, et on recristallise le résidu solide dans un mélange 95/5 d'éthanol et de méthanol.
Après filtration et séchage on obtient 1,3 g de fumarate (5:2)
Point de fusion : 190-192°C.

### Exemple 2A (Composé N°5A).

(*E*)-But-2-ènedioate de *exo*-8-[(4-chlorophényl)méthyl]*-N*-(2,3-dihydro-1*H*-indén-2-yl)-*N*-propyl-8-azabicyclo[3.2.1]. octane-3-méthanamine (2:1).

### 2A.1. exo-N-(2,3-dihydro-1H-indén-2-yl)-N-propyl-8-azabi cyclo[3.2.1]octane-3-méthanamine.

On prépare une suspension de 1,75 g (4,5 mmoles) de *exo-N*-(2,3-dihydro-1*H*-indén-2-yl)-8-(phénylméthyl)-*N*-propyl-8-azabicyclo[3.2.1]octane-3-méthanamine dans 40 ml d'éthanol, on ajoute 0,5 g de charbon palladié à 10% et on effectue une hydrogénation dans un appareil de Parr, sous une pression d'environ 0,32 MPa, à 45°C.

Après retour à la température ambiante on sépare le catalyseur par filtration, on concentre le filtrat sous pression réduite, on reprend le résidu huileux avec une solution d'ammoniaque et on l'extrait à l'éther diéthylique. On lave la phase organique à l'eau, on la sèche sur sulfate de sodium, on la filtre et on la concentre sous pression réduite.
On obtient 1,1 g de résidu huileux jaune qu'on utilise tel quel dans l'étape suivante.

### 2A.2. (E)-But-2-ènedioate de exo-8-[(4-chlorophényl)méthyl]-N-(2,3-dihydro-1H-indén-2-yl)-N-propyl-8-azabicyclo[3.2.1]octane-3-méthanamine (2:1).

Dans un ballon tricol de 100 ml, sous atmosphère d'argon, on introduit 2,94 g (9,8 mmoles) de *exo-N*-(2,3-dihydro-1*H*-indén-2-yl)-*N*-propyl-8-azabicyclo[3.2.1]octane-3-méthanamine en solution dans 45 ml de *N,N-*diméthylformamide, 2,2 g (10,7 mmoles) de 1-bromométhyl-4-chlorobenzène, 2,7 g (19,6 mmoles) de carbonate de potassium et 0,1 g d'iodure de sodium, puis on chauffe le mélange à 60°C pendant 3 h. On le laisse refroidir, on le verse sur 150 ml d'eau glacée et on l'extrait à l'acétate d'éthyle. On lave la phase organique à l'eau, on la sèche sur sulfate de sodium, on la filtre, on concentre le filtrat sous pression réduite et on purifie le résidu par chromatographie sur colonne de gel de silice en éluant avec un mélange 100/0 à 90/10 de dichlorométhane et de méthanol.
On obtient 4,0 g de base dont on prépare le fumarate par addition de 2,1 g (18,8 mmoles) d'acide fumarique en solution dans 50 ml d'éthanol aux 4,0 g (9,4 mmoles) de base en solution dans 100 ml d'éthanol, on évapore le solvant sous pression réduite, et on recristallise le résidu dans l'éthanol.
On obtient 2,62 g de solide blanc.
Point de fusion : 198-199°C.

### Exemple 3A (Composé N°3A).

Chlorhydrate de *exo*-8- (3-chlorobenzoyl)*-N-*(2,3-dihydro*-*1*H*-indén-2-yl)-*N*-propyl-8-azabicyclo[3.2.1]octane-3-méthanamine.

Dans un ballon tricol de 50 ml, sous atmosphère d'argon, on introduit 0,75 g (2,51 mmoles) de *exo-N*-(2,3-dihydro-1*H*-indén-2-yl)-*N*-propyl-8-azabicyclo[3.2.1]octane-3-méthanamine en solution dans 18 ml de dichlorométhane, on ajoute 0,51 g (5,03 mmoles) de triéthylamine puis, lentement, 0,88 g (5,02 mmoles) de chlorure de 3-chlorobenzoyle, et on agite le mélange à température ambiante pendant 24 h.
On verse le mélange sur 100 ml d'eau, on l'extrait à l'acétate d'éthyle, on sépare la phase organique, on la lave à l'eau, on la sèche sur sulfate de sodium, on la filtre, on concentre le filtrat sous pression réduite et on purifie le résidu par chromatographie sur colonne de gel de silice en éluant avec un mélange 99/1 à 98/2 de dichlorométhane et de méthanol.
On obtient 0,2 g de base dont on prépare le chlorhydrate par addition de 5 ml d'une solution 0,1 N d'acide chlorhydrique dans le propan-2-ol à une solution des 0,2 g (0,42 mmole) de base dans 10 ml d'acétate déthyle, on évapore les solvants sous pression réduite et on recristallise le résidu dans un mélange 95/5 d'acétate d'éthyle et de propan-2-ol.
On obtient 0,12 g de solide blanc.
Point de fusion : 213-215°C.

### Exemple 4A (Composé N°14A).

(*E*)-But-2-ènedioate de exo*-N*-(4,7-diméthoxy-2,3-dihydro-1*H*-indén-2-yl)-8-(phénylméthyl)-*N*-propyl-8-azabicyclo[3.2.1]. octane-3-méthanamine (2:1).

### 4A.1. exo-N-(4,7-diméthoxy-2,3-dihydro-1H-indén-2-yl)-8-(phénylméthyl)-8-azabicyclo[3.2.1]octane-3-carboxamide.

En utilisant le mode opératoire décrit dans l'exemple 1A.1, à partir de 4,64 g (24 mmoles) de 4,7-diméthoxy-2,3-dihydro-1*H*-indène-2-amine et 3,8 g (13,9 mmoles) de 8-(phénylméthyl)-8-azabicyclo[3.2.1]octane-3-carboxylate d'éthyle on obtient 4,1 g de solide qu'on utilise tel quel dans l'étape suivante.

### 4A.2. exo-N-(4,7-diméthoxy-2,3-dihydro-1H-indén-2-yl)-8-(phénylméthyl)-8-azabicyclo[3.2.1]octane-3-méthanamine.

En utilisant le mode opératoire décrit dans l'exemple 1A.2, à partir de 4,85 g (11,5 mmoles) de exo-N-(4,7-diméthoxy-2,3-dihydro-1*H*-indén-2-yl)-8-(phénylméthyl)-8-azabicyclo[3.2.1]octane-3-carboxamide et 0,88 g (23 mmoles) d'hydrure d'aluminium et de lithium, on obtient 4,6 g de produit huileux qu'on utilise tel quel dans l'étape suivante.

### 4A.3. exo-N-(4,7-diméthoxy-2,3-dihydro-1H-indén-2-yl)-N-[[8-(phénylméthyl)-8-azabicyclo[3.2.1]oct-3-yl]méthyl]propanamide.

En utilisant le mode opératoire décrit dans l'exemple 1A.3, à partir de 4,6 g (11,3 mmoles) de exo-N-(4,7-diméthoxy-2,3-dihydro-1*H*-indén-2-yl)-8-(phénylméthyl)-8-azabicyclo, [3.2.1]octane-3-méthanamine, 1,3 g (12,8 mmoles) de triéthylamine et 1,13 g (12,2 mmoles) de chlorure de propanoyle, on obtient, après purification par chromatographie sur colonne de gel de silice en éluant avec un mélange 98/2 à 92/8 de dichlorométhane et de méthanol, 4,7 g de produit huileux qu'on utilise tel quel dans l'étape suivante.

### 4A.4. (E)-But-2-ènedioate de exo-N-(4,7-diméthoxy-2,3-dihydro-1H-indén-2-yl)-8-(phénylméthyl)-N-propyl-8-azabicyclo[3.2.1]octane-3-méthanamine (2:1).

En utilisant le mode opératoire décrit dans l'exemple 1A.4, à partir de 4,7 g (10,1 mmoles) de *exo*-*N*-(4,7-diméthoxy-2,3-dihydro-1*H*-indén-2-yl) *-N-* [[8- (phénylméthyl)-8-azabicyclo[3.2.1]oct-3-yl]méthyl]propanamide et 0,78 g (20,6 mmoles) d'hydrure d'aluminium et de lithium, on obtient, après purification par chromatographie sur colonne de gel de silice en éluant avec un mélange 96/4 à 88/12 de dichlorométhane et de méthanol, 4,5 g de composé sous forme d'huile jaune.
On en dissout 1,0 g (2,23 mmoles) dans 100 ml d'éthanol, on ajoute une solution de 0,52 g (4,46 mmoles) d'acide fuma_ rique dans 100 ml d'éthanol, on évapore le solvant sous pression réduite, et on recristallise le résidu solide dans l'éthanol.
On obtient 0,68 g de fumarate (2:1).
Point de fusion : 187-189°C.

### Exemple 5A (Composé N°13A).

Chlorhydrate de *exo-N-*(4,7-diméthoxy-2,3-dihydro-1*H*-indén-2-yl)-8-(3-éthoxybenzoyl)-*N*-propyl-8-azabicyclo[3.2.1]. octane-3-méthanamine.

### 5A.1. exo-N-(4,7-diméthoxy-2,3-dihydro-1H-indén-2-yl)-N-propyl-8-azabicyclo[3.2.1]octane-3-méthanamine.

En utilisant le mode opératoire décrit dans l'exemple 2A.1, à partir de 4,95 g (11 mmoles) de *exo*-*N*-(4,7-diméthoxy-2,3-dihydro-1*H*-indén-2-yl)-8-(phénylméthyl)-*N*-propyl-8-azabicyclo[3.2.1]octane-3-méthanamine et 1,2 g de charbon palladié à 10%, on obtient 3,4 g de composé huileux qu'on utilise tel quel dans l'étape suivante.

### 5A.2. Chlorhydrate de exo-N-(4,7-diméthoxy-2,3-dihydro-1H-indén-2-yl)-8-(3-éthoxybenzoyl)-N-propyl-8-azabicyclo[3.2.1]octane-3-méthanamine.

En utilisant le mode opératoire décrit dans l'exemple 3A, à partir de 1,1 g (3,07 mmoles) de exo-*N*-(4,7-diméthoxy-2,3-dihydro-1*H*-indén-2-yl)*-N*-propyl-8-azabicyclo[3.2.1]octane-3-méthanamine, 0,62 g (6,14 mmoles) de triéthylamine et 1,13 g (6,12 mmoles) de chlorure de 3-éthoxybenzoyle, on obtient, après purification par chromatographie sur colonne de gel de silice en éluant avec un mélange 99/1 à 96/4 de dichlorométhane et de méthanol, 1,43 g de composé sous forme d'huile.
On prépare le chlorhydrate par addition de 30 ml d'acide chlorhydrique en solution 0,1 N dans le propan-2-ol à une solution de 1,43 g (2,82 mmoles) de base dans 30 ml d'éthanol, on évapore les solvants sous pression réduite, et on recristallise le résidu solide dans un mélange 9/1 d'acétate d'éthyle et d'éthanol.
On obtient 0,56 g de solide blanc.
Point de fusion : 161-163°C.

### Exemple 6A (Composé N°12A).

Chlorhydrate de *exo*-8-(3,4-diméthoxybenzoyl)*-N*-(4,7-diméthoxy-2,3-dihydro-1*H*-indén-2-yl)-*N*-propyl-8-azabicyclo[3.2.1]octane-3-méthanamine.

En utilisant le mode opératoire décrit dans l'exemple 3A, à partir de 1,25 g (3,49 mmoles) de *exo-N*-(4,7-diméthoxy-2,3-dihydro-1*H*-indén-2-yl)-*N*-propyl-8-azabicyclo[3.2.1]octane-3-méthanamine, 0,71 g (6,98 mmoles) de triéthylamine et 1,4 g (6,96 mmoles) de chlorure de 3,4-diméthoxybenzoyle, on obtient, après purification par chromatographie sur colonne de gel de silice en éluant avec un mélange 99/1 à 96,5/3,5 de dichlorométhane et de méthanol, 1,5 g de composé sous forme d'une huile jaune.
On prépare le chlorhydrate par addition de 30 ml d'acide chlorhydrique en solution 0,1 N dans le propan-2-ol à une solution de 1,5 g (2,87 mmoles) de base dans 30 ml d'éthanol, on évapore les solvants sous pression réduite, et on recristallise le résidu solide dans un mélange 9/1 d'acétate d'éthyle et d'éthanol.
On obtient 1,12 g de solide blanc.
Point de fusion : 134-136°C.

### Exemple 1B (Composé N°1B).

(*E*)-But-2-ènedioate de *exo*-*N*-[(2,3-dihydro-1,4-benzodioxan-2-yl)méthyl]-8-(phénylméthyl)-8-azabicyclo[3.2.1]octane-3-méthanamine (2:1).

### 1B.1. exo-N-[(2,3-dihydro-1,4-benzodioxan-2-yl)méthyl]-8-(phénylméthyl)-8-azabicyclo[3.2.1]octane-3-carbox amide.

Dans un ballon tricol de 500 ml on introduit, sous atmosphère d'azote, 20 ml de triméthylaluminium en solution 2 M dans le toluène, on refroidit le mélange à 0°C, on ajoute, goutte à goutte, 7,75 g (39,7 mmoles) de 2,3-dihydro-1,4-dioxane-2-méthanamine en solution dans 150 ml de toluène, on chauffe le mélange à 50°C puis, à cette température, on ajoute 6,9 g (25,1 mmoles) de 8-(phénylméthyl)-8-azabi cyclo[3.2.1]octane-3-carboxylate d'éthyle en solution dans 35 ml de toluène et on chauffe le mélange au reflux pendant 8 h.
On le refroidit à 0°C, on l'hydrolyse en ajoutant 50 ml d'eau, on le filtre sur terre d'infusoires, on sèche le filtrat-sur sulfate de sodium, on le filtre et on évapore les solvants sous pression réduite.
On purifie le résidu par chromatographie sur colonne de gel de silice en éluant avec un mélange 95/5 de dichlorométhane et de méthanol.
On obtient 8,4 g de composé sous forme d'huile.

### 1B.2. (E)-But-2-ènedioate de exo-N-[(2,3-dihydro-1,4-benzo. dioxan-2-yl)méthyl]-8-(phénylméthyl)-8-azabicyclo. [3.2.1]octane-3-méthanamine (2:1).

Dans un ballon tricol de 1 l, sous atmosphère d'azote, on introduit 5 g (131 mmoles) d'hydrure d'aluminium et de lithium en suspension dans 50 ml de tétrahydrofurane, on refroidit le mélange à 0°C, on ajoute 8,4 g (21,4 mmoles) de exo-*N*-[(2,3-dihydro-1,4-benzodioxan-2-yl)méthyl]-8-(phénylméthyl)-8-azabicyclo[3.2.1]octane-3-carboxamide en solution dans 420 ml de tétrahydrofurane et on chauffe le mélange au reflux pendant 15 h.
On le refroidit à 0°C, on hydrolyse l'excès d'hydrure avec de la soude aqueuse 1 N, on élimine l'insoluble par filtration, on sèche le filtrat sur sulfate de sodium et on le concentre sous pression réduite.
On obtient 7,3 g de base sous forme huileuse.
On en prélève 2,9 g (7,66 mmoles), on ajoute 1,8 g (15,5 mmoles) d'acide fumarique en solution dans l'éthanol, on évapore le solvant et on recristallise le résidu dans le propan-2-ol puis dans l'éthanol.
On obtient 1,3 g de solide blanc.
Point de fusion : 105-107°C.

### Exemple 2B (Composé N°15B).

Chlorhydrate de *exo*-*N*-[(2,3-dihydro-1,4-benzodioxan-2-yl)méthyl]-8-(4-méthoxybenzoyl)-8-azabicyclo[3.2.1]octane-3-méthanamine (1:1).

### 2B.1. exo-[(2,3-dihydro-1,4-benzodioxan-2-yl)méthyl] [[8-(phénylméthyl)-8-azabicyclo[3.2.1]oct-3-yl]méthyl]. carbamate de 1,1-diméthyléthyle.

Dans un ballon tricol, sous atmosphère d'azote, on introduit 7,3 g (193 mmoles) de *exo*-*N*-[(2,3-dihydro-1,4-benzodioxan-2-yl)méthyl]-8-(phénylméthyl)-8-azabicyclo[3.2.1]octane-3-méthanamine en solution dans 18 ml de dichlorométhane, on ajoute, goutte à goutte, 4,6 g (21 mmoles) de dicarbonate de bis(1,1-diméthyléthyle) en solution dans 18 ml de dichlorométhane, et on agite le mélange à température ambiante pendant 24 h.
On évapore le solvant sous pression réduite, on dissout le résidu dans 100 ml d'éther diéthylique, on lave la solution à l'eau, on la sèche sur sulfate de sodium, on la filtre et on concentre le filtrat sous pression réduite.
On obtient 8,8 g de produit brut qu'on purifie par chromatographie sur colonne de gel de silice en éluant avec un mélange 92,5/7,5 de dichlorométhane et de méthanol.
On obtient 7,7 g de composé sous forme d'huile.

### 2B.2. exo-[(2,3-dihydro-1,4-benzodioxan-2-yl)méthyl](8-azabicyclo[3.2.1]oct-3-ylméthyl)carbamate de 1,1-diméthyléthyle.

On prépare une solution de 7,4 g (15,45 mmoles) de *exo*-[(2,3-dihydro-1,4-benzodioxan-2-yl)méthyl][[8-(phénylméthyl)-8-azabicyclo[3.2.1]oct-3-yl]méthyl]carbamate de 1,1-diméthyléthyle dans 240 ml d'éthanol, on ajoute 3,5 g de charbon palladié à 10% et on effectue une hydrogénation dans un appareil de Parr à 35°C sous une pression de 0,30 MPa.
Après refroidissement à la température ambiante on sépare le catalyseur par filtration et on concentre le filtrat sous pression réduite.
On obtient 5,23 g de résidu huileux qu'on utilise tel quel dans l'étape suivante.

### 2B.3. exo-[(2,3-dihydro-1,4-benzodioxan-2-yl)méthyl] [[8-(4-méthoxybenzoyl)-8-azabicyclo[3.2.1]oct-3-yl]méthyl]carbamate de 1,1-diméthyléthyle.

Dans un ballon tricol de 250 ml, sous atmosphère d'azote, on introduit 2 g (5,15 mmoles) de *exo*-[(2,3-dihydro-1,4-benzodioxan-2-yl)méthyl](8-azabicyclo[3.2.1]oct-3-yl. méthyl)carbamate de 1,1-diméthyléthyle, 52 ml de *N,N*-diméthylformamide et 0,71 g (5,15 mmoles) de carbonate de potassium, on chauffe le mélange à 50°C, on ajoute 1 g (5,66 mmoles) de chlorure de 4-méthoxybenzoyle et on agite le mélange à 50°C pendant 10 h.
On évapore le solvant sous pression réduite, on ajoute au résidu 50 ml d'eau et 300 ml d'acétate d'éthyle, on sépare la phase organique, on la lave à l'eau, on la sèche sur sulfate de sodium, on la filtre, on concentre le filtrat sous pression réduite et on purifie le résidu par chromatographie sur colonne de gel de silice en éluant avec un mélange 92/8 de dichlorométhane et de méthanol.
On obtient 2,5 g de composé qu'on utilise tel quel dans l'étape suivante.

### 2B.4. Chlorhydrate de exo-N-[(2,3-dihydro-1,4-benzodioxan-2-yl)méthyl]-8-(4-méthoxybenzoyl)-8-azabicyclo. [3.2.1]octane-3-méthanamine (1:1).

Dans un ballon de 100 ml on introduit 2,5 g (48 mmoles) de *exo*-[(2,3-dihydro-1,4-benzodioxan-2-yl)méthyl][[8-(4-métho_ xybenzoyl)-8-azabicyclo[3.2.1]oct-3-yl]méthyl]carbamate de 1,1-diméthyléthyle, 25 ml de dichlorométhane et 25 ml d'acide trifluoroacétique et on chauffe le mélange au reflux pendant 8 h.
On le refroidit, on ajoute, goutte à goutte, 30 ml de solution aqueuse de soude 10 N, on sépare la phase aqueuse, on l'extrait au dichlorométhane, on lave la phase organique à l'eau, on la sèche sur sulfate de sodium, on la filtre, on concentre le filtrat sous pression réduite et on purifie le résidu par chromatographie sur colonne de gel de silice en éluant avec un mélange 96/4 de dichlorométhane et de méthanol.
On obtient 0,85 g de base qu'on transforme en chlorhydrate par addition d'éther diéthylique saturé d'acide chlorhydrique gazeux.
On obtient 0,45 g de solide blanc.
Point de fusion : 93-110°C.

### Exemple 3B (Composé N°12B).

Chlorhydrate de *exo-N*-[(2,3-dihydro-1,4-benzodioxan-2-yl)méthyl]-8-(3-fluorobenzoyl)-8-azabicyclo[3.2.1]octane-3-méthanamine (1:1).

### 3B.1. exo-[(2,3-dihydro-1,4-benzodioxan-2-yl)méthyl][[8-(3-fluorobenzoyl)-8-azabicyclo[3.2.1]oct-3-yl]méthyl] carbamate de 1,1-diméthyléthyle.

En utilisant le mode opératoire décrit dans l'exemple 2B.3, à partir de 3,5 g (9 mmoles) de *exo*-[(2,3-dihydro-1,4-benzodioxan-2-yl)méthyl](8-azabicyclo[3.2.1]oct-3-ylméthyl)carbamate de 1,1-diméthyléthyle et 1,57 g (99 mmoles) de chlorure de 3-fluorobenzoyle, en présence d'une quantité catalytique d'iodure de potassium, on obtient 2,7 g de produit huileux qu'on utilise tel quel dans l'étape suivante.

### 3B.2. Chlorhydrate de exo-N-[(2,3-dihydro-1,4-benzodioxan-2-yl)méthyl]-8-(3-fluorobenzoyl)-8-azabicyclo[3.2.1]octane-3-méthanamine (1:1).

En utilisant le mode opératoire décrit dans l'exemple 2B.4, à partir de 2,7 g (5,28 mmoles) de exo-[(2,3-dihydro-1,4-benzodioxan-2-yl)méthyl] [[8-(3-fluorobenzoyl)-8-azabicyclo [3.2.1]oct-3-yl]méthyl]carbamate de 1,1-diméthyléthyle, on obtient, après purification par chromatographie sur colonne de gel de silice en éluant avec un mélange 96,5/3,5 de dichlorométhane et de méthanol, 1,6 g de composé sous forme de base.
On prépare le chlorhydrate en traitant 0,5 g de base avec une solution 0,1 N d'acide chlorhydrique dans le propan-2-ol, on évapore le solvant et on recristallise le résidu dans l'acétate d'éthyle.
On isole finalement 0,4 g de solide blanc.
Point de fusion : 206-209°C.

### Exemple 4B (Composé N°4B).

(*E*)-But-2-ènedioate de *exo-N-* [(2,3-dihydro-1,4-benzodioxan-2-yl)méthyl]-8- (3-fluorophényl) -8-azabicyclo[3.2.1]octane-3-méthanamine (1:1).

Dans un ballon tricol de 250 ml, sous atmosphère d'azote, on introduit 0,25 g (6,6 mmoles) d'hydrure d'aluminium et de lithium, 10 ml de tétrahydrofurane et 1 g (2,4 mmoles) de *exo-N*-[(2,3-dihydro-1,4-benzodioxan-2-yl)méthyl]-8-(3-fluorobenzoyl)-8-azabicyclo[3.2.1]octane-3-méthanamine en solution dans 60 ml de tétrahydrofurane, et on agite le mélange à la température du reflux pendant 2 h, puis à température ambiante pendant 24 h.
On le refroidit à 0°C, on hydrolyse l'excès d'hydrure avec de la soude aqueuse 1 N, on élimine l'insoluble par filtration, on sèche le filtrat sur sulfate de sodium, on le filtre, on concentre le filtrat sous pression réduite et on purifie le résidu par chromatographie sur colonne de gel de silice en éluant avec un mélange 93/7 de dichlorométhane et de méthanol.
On obtient 0,6 g de base qu'on dissout dans 5 ml d'éthanol, on ajoute 0,35 g (3 mmoles) d'acide fumarique et on laisse cristalliser par refroidissement.
On obtient 0,2 g de fumarate.
Point de fusion : 132-134°C.

### Exemple 5B (Composé N°8B).

(*E*)-But-2-ènedioate de *exo-N*-[(2,3-dihydro-1,4-benzodioxan-2-yl)méthyl]-8- (phénylméthyl) -*N*-propyl-8-azabicyclo[3.2.1]-octane-3-méthanamine (2:1).

### 5B.1. exo-N- [(2,3-dihydro-1,4-benzodioxan-2-yl)méthyl]-N-[[8-(phénylméthyl)-8-azabicyclo[3.2.1]oct-3-yl]méthyl]propanamide.

Dans un ballon tricol de 250 ml on introduit 3 g (7,9 mmoles) de *exo-N-* [(2,3-dihydro-1,4-benzodioxan-2-yl)méthyl]-8-(phénylméthyl)-8-azabicyclo[3.2.1]octane-3-méthanamine, 100 ml de dichlorométhane, 0,75 g (8,7 mmoles) de chlorure de propanoyle en solution dans 10 ml de dichlorométhane, et on agite le mélange à température ambiante pendant 20 h.
On lave le mélange avec trois fois 50 ml d'eau, on sèche la phase organique sur sulfate de sodium, on la filtre, on concentre le filtrat sous pression réduite, et on purifie le résidu par chromatographie sur gel de silice en éluant avec un mélange 94/6 de dichlorométhane et de méthanol.
On obtient 1,6 g de composé qu'on utilise tel quel dans l'étape suivante.

### 5B.2. (E)-But-2-ènedioate de exo-N- [(2,3-dihydro-1,4-benzodioxan-2-yl)méthyl]-8-(phénylméthyl)-N-propyl-8-azabicyclo[3.2.1]octane-3-méthanamine (2:1).

Dans un ballon tricol de 100 ml, sous atmosphère d'azote, on introduit 0,3 g (7,4 mmoles)d'hydrure d'aluminium et de lithium et 15 ml de tétrahydrofurane, on refroidit le mélange à 0°C, on ajoute 1,6 g (3,7 mmoles) de *exo-N-*[(2,3-dihydro-1,4-benzodioxan-2-yl)méthyl]-N-[[8-(phénylméthyl)-8-azabicyclo[3.2.1]oct-3-yl]méthyl]propanamide en solution dans 32 ml de tétrahydrofurane, et on chauffe le mélange au reflux pendant 7 h.
On le refroidit à 0°C, on hydrolyse l'excès d'hydrure avec de la soude aqueuse 1 N, on élimine l'insoluble par filtration, on sèche le filtrat sur sulfate de sodium, on le filtre et on concentre le filtrat sous pression réduite. On prépare le fumarate à partir de 1,45 g (3,44 mmoles) de base en solution dans 5 ml d'éthanol et 0,8 g (6,9 mmoles) d'acide fumarique en solution dans 10 ml d'éthanol. On recueille le précipité par filtration et on le recristallise dans l'éthanol.
On obtient 1,1 g de solide blanc.
Point de fusion : 190-191°C.

### Exemple 6B (Composé N°21B).

Chlorhydrate de *exo-N*-[(2,3-dihydro-1,4-benzodioxan-2-yl)méthyl]-8-(4-méthylbenzoyl)-*N*-propyl-8-azabicyclo[3.2.1]octane-3-méthanamine (1:1).

### 6B.1. exo-N- [(2,3-dihydro-1,4-benzodioxan-2-yl)méthyl] -N-propyl-8-azabicyclo[3.2.1]octane-3-méthanamine.

Dans un ballon de 250 ml, sous atmosphère d'azote, on introduit 3,45 g (8,2 mmoles) de *exo*-*N*-[(2,3-dihydro-1,4-benzodioxan-2-yl)méthyl]-8-(phénylméthyl)-*N*-propyl-8-azabicyclo[3.2.1]octane-3-méthanamine, 70 ml de méthanol, 3,4 g de charbon palladié à 10% et 3,4 g de formiate d'ammonium, et on chauffe le mélange au reflux pendant 2 h. On le laisse refroidir, on élimine le catalyseur par filtration, on évapore le solvant sous pression réduite, on dissout le résidu dans du dichlorométhane, on lave la solution à l'eau, on la sèche sur sulfate de sodium, on la filtre et on concentre le filtrat sous pression réduite.
On obtient 1,8 g de composé qu'on utilise tel quel dans l'étape suivante.

### 6B.2. Chlorhydrate de exo-N- [(2,3-dihydro-1,4-benzodioxan-2-yl)méthyl]-8-(4-méthylbenzoyl)-N-propyl-8-azabi. cyclo[3.2.1]octane-3-méthanamine (1:1).

Dans un ballon tricol de 100 ml, sous atmosphère d'azote, on introduit 1 g (3 mmoles) de *exo*-*N*-[(2,3-dihydro-1,4-benzodioxan-2-yl)méthyl]-*N*-propyl-8-azabicyclo[3.2.1]. octane-3-méthanamine, 10 ml de tétrahydrofurane, 10 ml d'acétate d'éthyle et 0,31 g (3 mmoles) de triéthylamine. On refroidit le mélange à 0°C, on ajoute, goutte à goutte, 0,51 g (3,3 mmoles) de chlorure de 4-méthylbenzoyle en solution dans 1 ml d'acétate d'éthyle et on agite le mélange à température ambiante pendant 5 h.
On élimine le chlorhydrate de triéthylamine par filtration, on concentre le filtrat sous pression réduite, on dissout le résidu dans du dichlorométhane, on lave la solution à l'eau, on la sèche sur sulfate de sodium, on la filtre, et on concentre le filtrat sous pression réduite.
On obtient 1,4 g de base dont on prépare le chlorhydrate par addition de 30 ml d'éther diéthylique saturé d'acide chlorhydrique gazeux.
Après recristallisation on obtient 0,65 g de solide blanc.
Point de fusion : 95-112°C.

### Exemple 7B (Composé N°22B).

Chlorhydrate de 8-benzoyl*-N*-[(3,4-dihydro-2*H*-1-benzopyran-2-yl)méthyl]-8-azabicyclo[3.2.1]octane-3-méthanamine.

On prépare une suspension de 0,6 g (1,9 mmoles) de 4-méthylbenzènesulfonate de (3,4-dihydro-2*H*-1-benzopyran-2-yl)méthyle, 1 g (4,1 mmoles) de 8-benzoyl-8-azabicyclo[3.2.1]octane-3-méthanamine et 0,26 g (1,9 mmoles) de carbonate de potassium dans 11 ml d'acétonitrile, et on la chauffe au reflux pendant 18 h.
On ajoute un peu d'iodure de potassium et 0,15 g de carbonate de potassium supplémentaire et on chauffe au reflux pendant encore 8 h.
On élimine l'insoluble par filtration, on concentre le filtrat sous pression réduite, on reprend le résidu avec du dichlorométhane, on lave la solution à l'eau, on la sèche sur sulfate de sodium, on la filtre, on concentre le filtrat sous pression réduite et on purifie le résidu par chromatographie sur colonne de gel de silice en éluant avec un mélange 80/20 puis 70/30 d'acétate d'éthyle et de méthanol.
On prépare le chlorhydrate au moyen d'une solution 0,1 N d'acide chlorhydrique dans le propan-2-ol.
On obtient 0,4 g de solide.
Point de fusion : 148,5-151,5°C.

### Exemple 8B (Composé N°24B).

Chlorhydrate de 8-benzoyl*-N*-[(4-oxo-2,3-dihydro-4*H*-1-benzopyran-3-yl)méthyl]-8-azabicyclo[3.2.1]octane-3-méthanamine.

Dans un ballon tricol on introduit 1,71,g (6,1 mmoles) de chlorhydrate de 8-benzoyl-8-azabicyclo[3.2.1]octane-3-méthanamine, 0,2 g (6,3 mmoles) de paraformaldéhyde et quelques gouttes d'acide chlorhydrique concentré dans 10 ml de propan-2-ol, on chauffe le mélange au reflux pendant 5 min, on ajoute 0,78 g (5,3 mmoles) de 2,3-dihydro-4*H*-1-benzopyran-4-one en solution dans 10 ml de propan-2-ol et on reprend le chauffage au reflux pendant 18 h.
On refroidit le mélange et on recueille directement le chlorhydrate par filtration.
On obtient 1,26 g de solide.
Point de fusion : 187-189°C.

### Exemple 9B (Composé N°29B).

Chlorhydrate de (S)-*exo*-8-benzoyl-*N*-[8-méthoxy-2,3-dihydro-1,4-benzodioxan-2-yl)méthyl]-8-azabicyclo[3.2.1]octane-3-méthanamine (1:1).

### 9B.1. (S)-8-méthoxy-1,4-benzodioxane-2-méthanol. La préparation de ce composé est décrite dans Tet. Letters (1992) 33 6283-6286.

### 9B.2. (R)-4-Méthylbenzènesulfonate de (8-méthoxy-2,3-dihydro-1,4-benzodioxan-2-yl)méthyle.

On dissout 410 mg (2,1 mmoles) de (*S*)-8-méthoxy-1,4-benzodioxane-2-méthanol dans 5 ml de pyridine, on ajoute 398 mg (2,1 mmoles) de chlorure de 4-méthylbenzènesulfonyle et on agite le mélange à température ambiante pendant 20 h. On le verse sur 30 ml d'eau glacée, on l'extrait avec 2 fois 15 ml d'acétate d'éthyle, on lave la phase organique avec une solution aqueuse 1 N d'acide chlorhydrique, puis avec une solution aqueuse d'hydrogénocarbonate de sodium, puis avec de l'eau, et on la sèche sur sulfate de sodium. On la filtre, on évapore le solvant sous pression réduite, on reprend le résidu avec du pentane, on recueille le solide gommeux, on le lave au pentane et on le sèche sous pression réduite.
On isole 405 mg de composé qu'on utilise tel quel dans l'étape suivante.

### 9B.3. Chlorhydrate de (S)-exo-8-benzoyl-N-[8-méthoxy-2,3-dihydro-1,4-benzodioxan-2-yl)méthyl]-8-azabicyclo[3.2.1]octane-3-méthanamine (1:1).

Sous atmosphère inerte on prépare une suspension de 380 mg (1,08 mmole) de (*R*)-4-méthylbenzènesulfonate de (8-méthoxy-2,3-dihydro-1,4-benzodioxan-2-yl)méthyle, 530 mg (2,16 mmoles) de 8-benzoyl-8-azabicyclo[3.2.1]octane-3-méthanamine et 180 mg (1,3 mmole) de carbonate de potassium dans 5 ml d'acétonitrile, et on la chauffe au reflux pendant 57 h.
On évapore le solvant sous pression réduite, on reprend le résidu avec du dichlorométhane et de l'eau, on sépare la phase organique, on la lave à l'eau jusqu'à pH neutre, on la filtre, on évapore le solvant sous pression réduite, et on purifie le résidu par chromatographie sur colonne de gel de silice en éluant avec un mélange 98/2 puis 95/5 de dichlorométhane et de méthanol.
On prépare le chlorhydrate au moyen d'une solution 0,1 N d'acide chlorhydrique dans le propan-2-ol.
On isole finalement 135 mg de solide.
Point de fusion : 210-211°C [α]_{D}²⁰= -53° (c=0,1, MeOH).

### Exemple 10B (Composé N°31B).

Chlorhydrate de (*S*)-exo-8-benzoyl-*N*-[7-chloro-2,3-dihydro-1,4-benzodioxan-2-yl)méthyl]-8-azabicyclo[3.2.1]octane-3-méthanamine (1:1).

### 10B.1. (R)-4-Méthylbenzènesulfonate de (7-chloro-2,3-dihydro-1,4-benzodioxan-2-yl)méthyle.

La préparation de ce composé est décrite dans la demande de brevet WO 97/03071, pages 42-43.

### 10B.2. Chlorhydrate de (S)-exo-8-benzoyl-N-[7-chloro-2,3-dihydro-1,4-benzodioxan-2-yl)méthyl]-8-azabicyclo[3.2.1]octane-3-méthanamine (1:1).

On prépare une suspension de 1,1 g (3,1 mmoles) de (*R*)-4-méthylbenzènesulfonate de (7-chloro-2,3-dihydro-1,4-benzodioxan-2-yl)méthyle, 1,5 g (6,2 mmoles) de 8-benzoyl-8-azabicyclo[3.2.1]octane-3-méthanamine et 515 mg (3,7 mmoles) de carbonate de potassium dans 20 ml d'acéto. nitrile, et on la chauffe au reflux pendant 48 h.
On évapore le solvant sous pression réduite, on reprend le résidu avec du dichlorométhane et de l'eau, on sépare la phase organique, on la lave jusqu'à pH neutre, on la sèche sur sulfate de sodium, on la filtre, on évapore le filtrat sous pression réduite, et on purifie le résidu par chromatographie sur colonne de gel de silice en éluant avec un mélange 98/2 puis 95/5 de dichlorométhane et de méthanol.
On prépare le chlorhydrate au moyen d'une solution 0,1 N d'acide chlorhydrique dans le propan-2-ol.
On isole finalement 230 mg de solide.
Point de fusion : 164,5-167°C [α]_{D}²⁰= -56° (c=0,1, MeOH).

Les tableaux A et B qui suivent illustrent les structures chimiques et les propriétés physiques de quelques composés de l'invention.

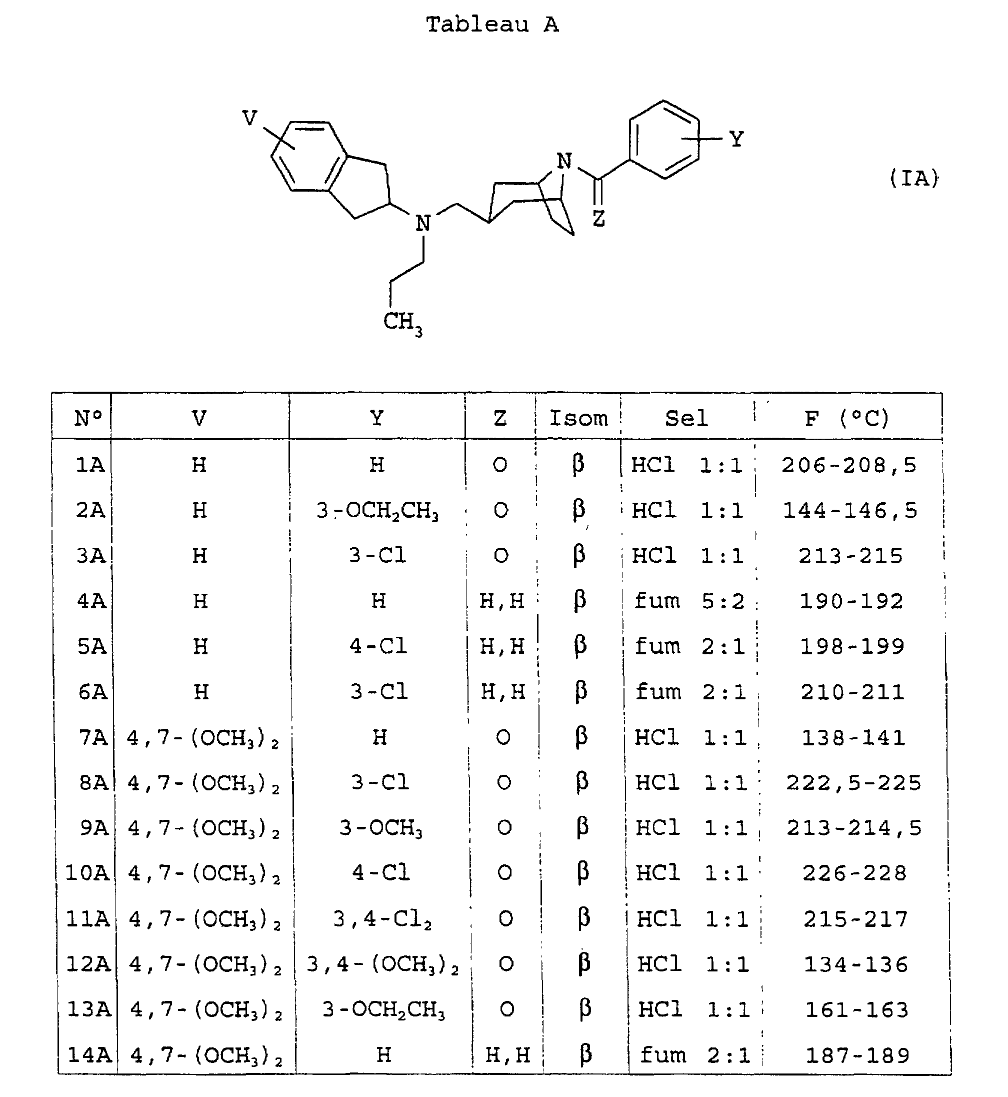

Dans la colonne "Isom" est indiquée la forme isomère géométrique du cycle azabicyclooctane.
Dans la colonne "Sel", "HCl" désigne un chlorhydrate et "fum" désigne un (*E*)-but-2-ènedioate, ou fumarate ; les rapports molaires acide:base sont indiqués.

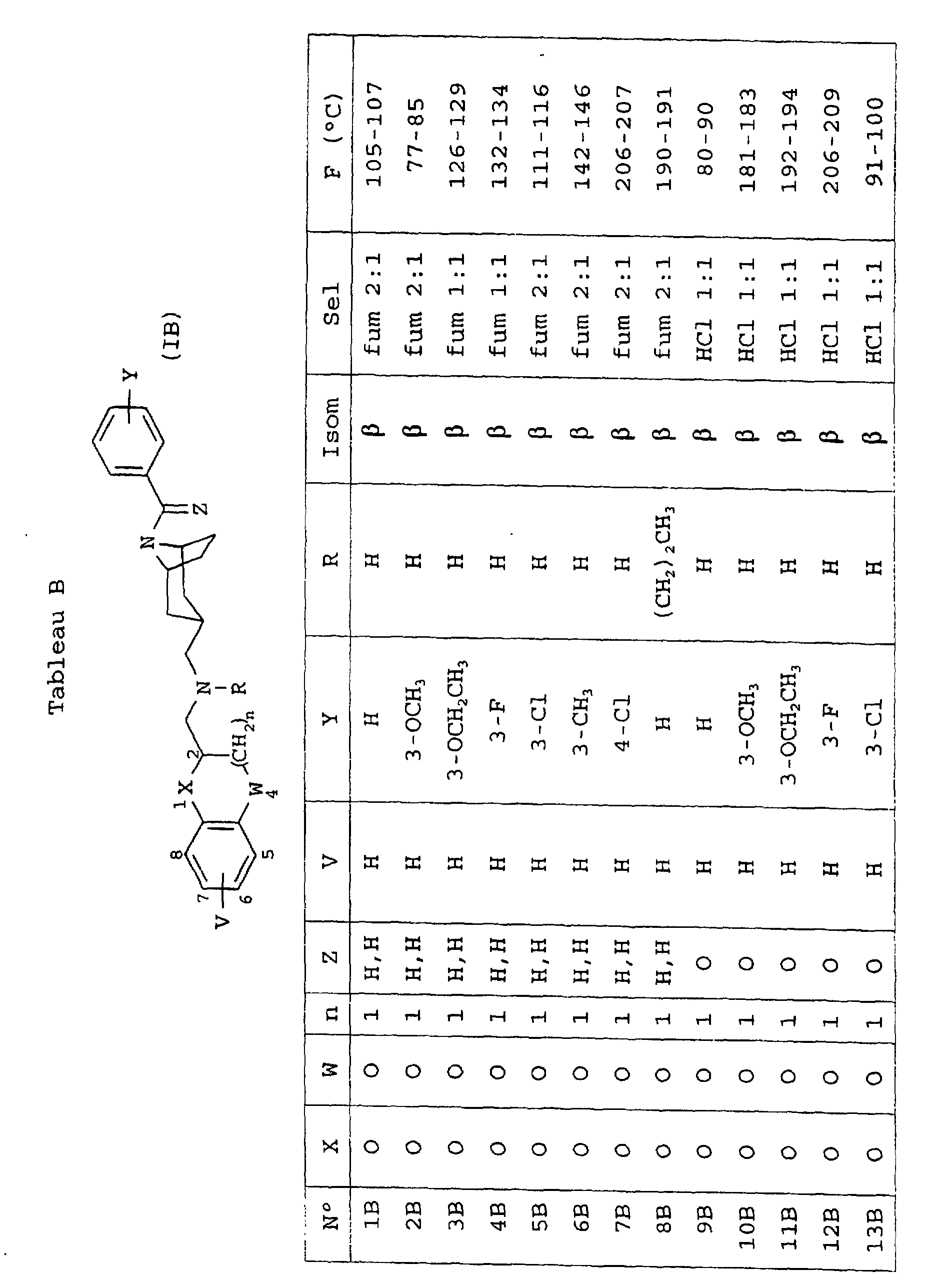

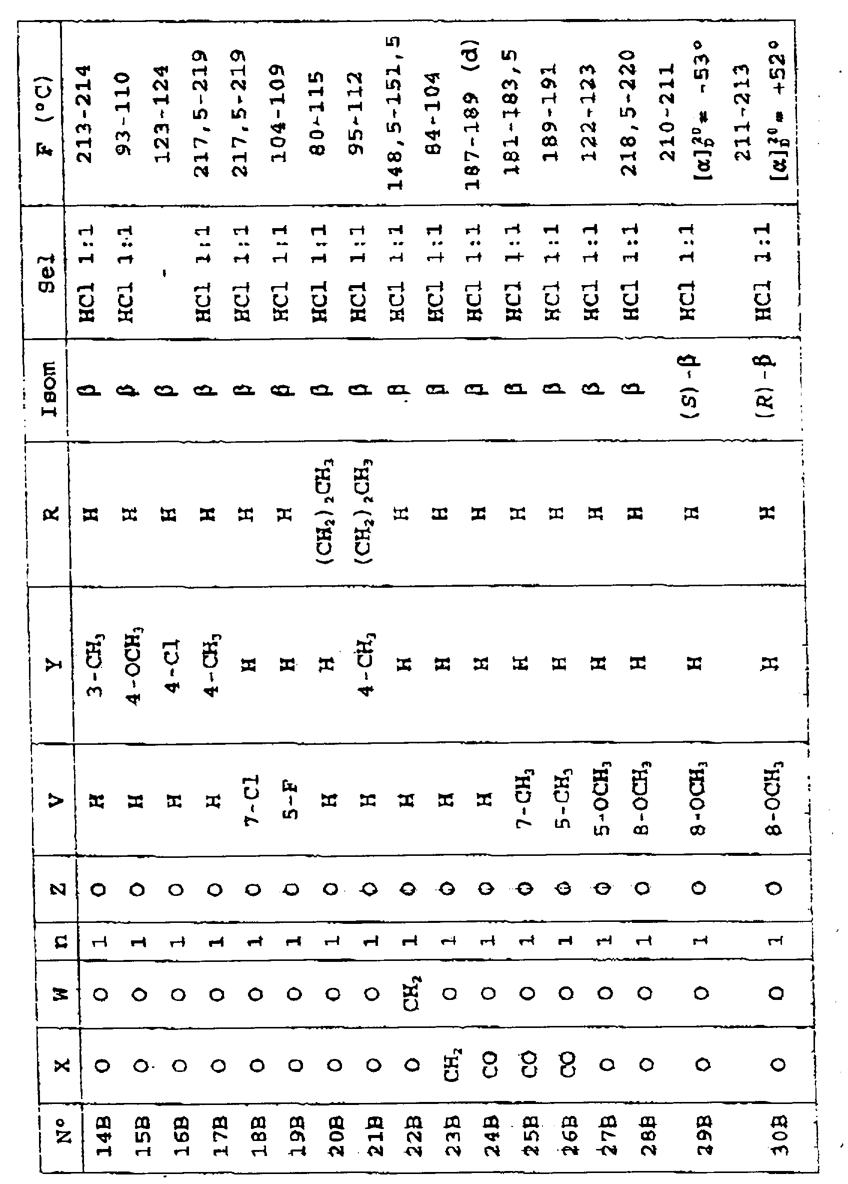

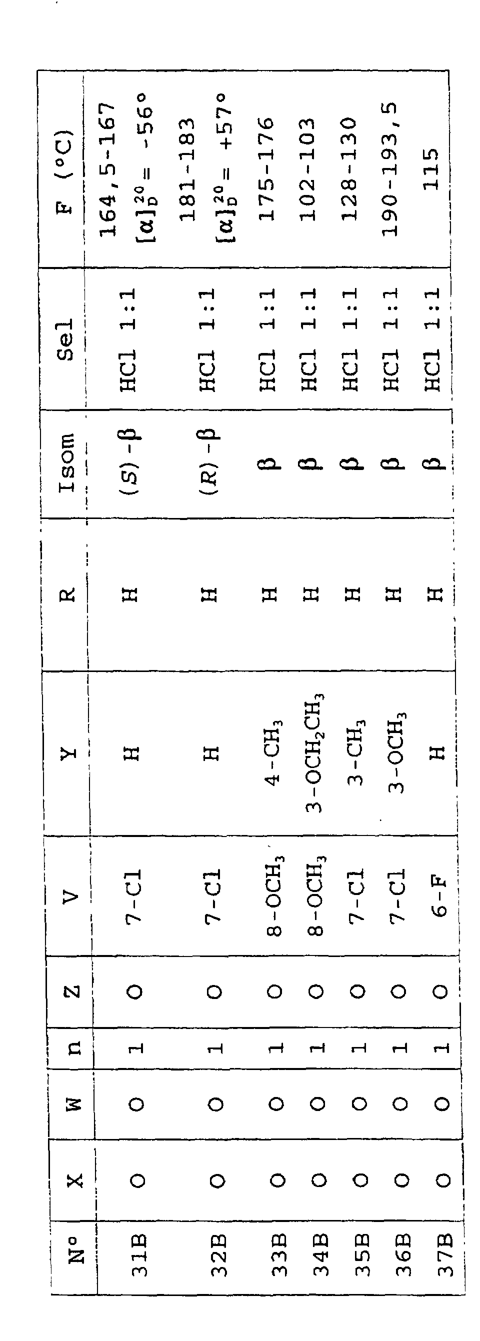

La numérotation des atomes de l'hétérocycle contenant W et X est données à titre indicatif, essentiellement pour situer le substituant V ; elle n'est pas nécessairement conforme aux règles de la nomenclature, en particulier lorsque X représente un groupe CH₂ ou CO.
Dans la colonne "Isom" est indiquée la forme isomère géométrique du cycle azabicyclooctane. Les mentions (*R*) et (*S*) concernent l'atome en position 2 du cycle 2,3-dihydro-1,4-dioxane.
Dans la colonne "Sel", "-" désigne un composé à l'état de base, "HCl" désigne un chlorhydrate et "fum" désigne un (*E*)-but-2-ènedioate, ou fumarate ; les rapports molaires acide:base sont indiqués.
Dans la colonne "F (°C)", "(d)" indique un point de fusion avec décomposition.
Les pouvoirs rotatoires des composés 29B à 32B sont donnés pour (c=0,1, MeOH).

Les composés de l'invention ont été soumis a une série d'essais pharmacologiques qui ont mis en évidence leur intérêt comme substances à activités thérapeutiques.

### Etude de l'affinité pour les récepteurs dopaminergiques du type D₂ dans le striatum de rat.

Les composés déplacent la liaison d'un ligand spécifique marqué, la spipérone (désignée ci-après par "[³H]spipérone" et décrite par Briley et Langer., *Eur. J. Pharmacol.* (1978) **50** 283) sur les récepteurs D₂ présents dans le striatum du rat.
Les animaux utilisés sont des rats mâles Sprague-Dawley de 150 à 250 g. Après décapitation on en prélève le cerveau et on excise le striatum. On broie le tissu à l'aide d'un broyeur Polytron™ dans 50 volumes de tampon Tris-HCl 50 mM contenant du chlorure de sodium (120 mM), du chlorure de potassium (5 mM) et dont le pH est ajusté à 7,4 (soit 100 mg de tissu frais par 5 ml). On lave les tissus homogénéisés deux fois à 4°C, en les centrifugeant à chaque fois pendant 10 mn à 40000×g et en remettant le culot en suspension dans du tampon frais refroidi. Finalement on met le dernier culot en suspension dans le même volume de tampon et on ajoute de l'acide ascorbique (0,1 % en concentration finale) et de la pargyline (10 µM en concentration finale). On laisse ensuite incuber à 37°C pendant 10 mn.
On détermine la liaison de la [³H]spipérone (New England Nuclear, activité spécifique 20-40 mCi/mmole) en faisant incuber 100 µl de la suspension membranaire avec le radio-ligand (0,25 nM) dans un volume final de 1 ml, pendant 20 minutes, à 37°C, en présence ou en l'absence du composé à étudier. On détermine la liaison non spécifique en présence d'halopéridol à la concentration de 10 µM. Après incubation, on récupère les membranes par filtration sur filtres Whatman GF/B™ qu'on lave avec deux volumes de 5 ml de tampon glacé. On extrait les filtres dans le liquide de scintillation et on en mesure la radioactivité par scintigraphie liquide avec une efficacité de 50 à 60 %. Pour chaque composé testé, on exprime les résultats par la CI₅₀, c'est à dire par la concentration qui inhibe 50 % de la liaison de la [³H]spipérone, calculée par une méthode graphique ou mathématique.
Pour les composés de l'invention les CI₅₀ se situent entre 0,005 et 2 µM.

### Etude de l'affinité pour les récepteurs dopaminergiques D₃ dans le noyau caudé bovin.

Les composés ont fait l'objet d'une étude *in vitro* quant à leur affinité pour les récepteurs dopaminergiques D₃ obtenus à partir d'une préparation membranaire du noyau caudé bovin, essentiellement comme décrit par Schoemaker H. dans *Eur. J. Pharmacol.* (1993) **242** R1-R2.
Le jour de l'expérience, les noyaux caudés bovins (Collect Organe, Paris, France), entreposés à -80°C, sont décongelés et homogénéisés à 4°C dans 10 volumes de tampon (Tris 10 mM, EDTA 1 mM, pH 7,5 à 25°C) à l'aide d'un Polytron™ (position 5, 30 s). L'homogénat est centrifugé à 2500 g pendant 1 min (centrifugeuse Sorvall™ munie d'un rotor SS34). Le surnageant est récupéré et centrifugé à 35000 g pendant 15 min, le culot est lavé par remise en suspension dans 10 volumes de tampon, homogénéisation et centrifugation, et le culot final est mis en suspension dans 10 volumes de tampon et préincubé à 37°C pendant 10 min.
L'homogénat est centrifugé à 35000 g pendant 15 min, le culot est remis en suspension dans le tampon d'incubation, (HEPES 50 mM, EDTA 1 mM, 8-hydroxyquinoléine 50 µM, acide ascorbique 0,005%, pH 7,5 à 25°C), à raison de 100 mh de tissu initial par ml.
La suspension membranaire (150 µl) est incubée à 23°C pendant 60 min dans des tubes, en présence de 0,8 nM de [³H]7-OH-DPAT (activité spécifique 120-160 Ci/mmole, Amersham™) dans un volume final de 1 ml de tampon d'incubation contenant 0,2 µM de chlorhydrate de zolpidem et 1 mg d'albumine de serum bovin, en présence ou en l'absence de composé à tester. L'incubation est arrêtée par filtration sur Brandel Harvester M-48™, avec utilisation de filtres Whatman GF/C™ préalablement traités avec de l'albumine de serum bovin (0,1% pendant 30 min. Après prédilution avec 4 ml de tampon (Tris 50 mM, NaCl 120 mM, KCl 5 mM, pH 7,4 à 25°C) de chaque milieu réactionnel, les tubes sont rincés 2 fois avec 4 ml de ce tampon.
Les filtres sont découpés puis séchés dans une étuve à 120°C pendant 10 min et la radioactivité retenue sur les filtres est déterminée par spectrométrie à scintillation liquide. La liaison non spécifique est déterminée en présence de 1 µM de dopamine.
Pour chaque concentration de composé étudié, le pourcentage d'inhibition de la liaison spécifique du [³H]7-OH-DPAT est calculée, puis la CI₅₀, concentration qui inhibe 50% de la liaison, est déterminée.
Les CI₅₀ des composés de l'invention sont de l'ordre de 0,005 à 2 µM.

### Etude de l'affinité pour les récepteurs sérotoninergiques du type 5-HT_{1A}.

Les composés déplacent la liaison d'un ligand spécifique marqué, la [³H]-8-hydroxy-di-n-propylamino-2 tétraline (désignée ci-après par "[³H]-8-OH-DPAT" et décrite par Gozlan et coll., *Nature* (1983) **305** 140) sur les récepteurs 5-HT_{1A} présents dans l'hippocampe du rat.
Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron™ pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés deux fois à 4°C, en les centrifugeant à chaque fois pendant 10 mn à 48000×g et en remettant le culot en suspension dans du tampon frais refroidi. Finalement on met le dernier culot en suspension dans le tampon pour arriver à une concentration de 50 mg de tissu de départ par ml de tampon à 50 mM. On laisse ensuite incuber à 37°C pendant 10 mn.
On détermine la liaison avec la [³H]8-OH-DPAT (1 nM) par incubation de 50 µl de suspension de membranes dans un volume final de 250 µl de tampon contenant 10 µM de pargyline et 3 µM de paroxétine. Après une incubation de 15 mn à 37°C on récupère les membranes par filtration sur filtres Whatman GF/B™ qu'on lave trois fois avec des quantités aliquotes de 5 ml de tampon glacé. On extrait les filtres dans le liquide de scintillation et on en mesure la radioactivité par scintigraphie liquide. On définit la liaison spécifique de la [³H]8-OH-DPAT comme la quantité de radioactivité retenue sur les filtres et pouvant être inhibée par co-incubation avec de la 5-hydroxytryptamine à 10 µM. A une concentration de 1 nM de [³H]8-OH-DPAT la liaison spécifique représente 90% de la radioactivité totale récupérée sur le filtre.
Pour chaque concentration de composé étudié on détermine le pourcentage d'inhibition de la liaison avec la [3H]8-OH-DPAT, puis la concentration CI₅₀, concentration qui inhibe 50% de la liaison.
Pour les composés de l'invention les CI₅₀ se situent entre 0,5 et 500 nM.

### Etude de l'affinité pour les récepteurs sérotoninergiques de type 5-HT₂.

Les composés de l'invention ont encore fait l'objet d'un essai *in vitro* de déplacement de la liaison de la spipérone sur les récepteurs sérotoninergiques (5-HT₂) du cortex cérébral du rat.
Pour cet essai on prélève les cerveaux de rats, on en dissèque le cortex et on l'homogénéise à 0°C dans 10 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000×g pendant 10 min puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 100 mg de tissu humide pour 1 ml de tampon.
On soumet alors le tissu à une incubation préalable de 10 min à 37°C en présence de 10 micromoles/l de pargyline, puis à une incubation de 20 min à 37°C en présence de [³H]spipérone (activité spécifique : 15 à 30 Ci par millimole) à la concentration de 0,3 nanomole/l et du composé à étudier.
On récupère ensuite les membranes par filtration sur filtres Whatman GF/B™, qu'on lave deux fois avec 5 ml de tampon froid. La radioactivité retenue par le filtre est mesurée par scintigraphie liquide.
Pour évaluer l'activité des composés on établit la courbe du pourcentage d'inhibition de la liaison spécifique de ^{[3}H]spipérone en fonction de la concentration en drogue déplaçante. On détermine graphiquement la concentration CI₅₀, concentration qui inhibe 50 % de la liaison spécifique.
La liaison spécifique est définie comme étant la liaison déplacée par 100 micromoles/l de 5-HT.
Les concentrations CI₅₀ des composés de l'invention se situent entre 0,2 et 5 µM.

Les résultats des essais montrent que les composés de l'invention possèdent une forte affinité pour les récepteurs dopaminergiques de type D₂ et D₃ et pour les récepteurs sérotoninergiques de type 5-HT_{1A} et 5-HT₂.
Ces résultats suggèrent que les composés peuvent être utilisés pour le traitement des affections et pathologies liées aux dysfonctionnements des transmissions dopamin-ergique et sérotoninergique, en particulier des récepteurs dopaminergiques D₂ et D₃ et sérotoninergiques 5-HT_{1A} et 5-HT₂.
Ainsi on peut les utiliser pour le traitement des psychoses, en particulier de la schizophrénie (forme déficitaire et forme productive) et des symptômes extrapyramidaux aigus ou chroniques induits par les neuroleptiques, pour le traitement des diverses formes d'anxiété, des attaques de panique, des phobies, des troubles obsessionnels compulsifs, pour le traitement des différentes formes de dépression, y compris la dépression psychotique, pour le traitement des troubles dus à l'abus ou au sevrage d'alcool, des troubles du comportement sexuel, des troubles de la prise de nourriture, et pour le traitement de la migraine.
A cet effet ils peuvent être présentés sous toutes formes appropriées à leur administration par voie orale ou parentérale, associés à tous excipients convenables, et dosés pour permettre une posologie journalière de 1 à 1000 mg.

## Revendications

1. Composé, sous forme d'isomère géométrique pur ou de mélange de tels isomères, répondant à la formule générale (I) 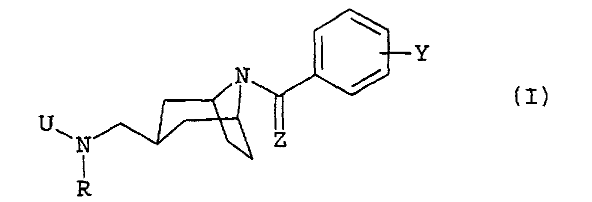 dans laquelle
U représente
A) soit un groupe 2,3-dihydro-1*H*-indén-2-yle de formule générale (A) 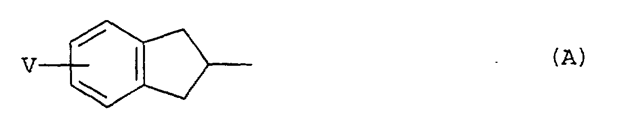
B) soit un groupe hétérocyclique de formule générale (B) 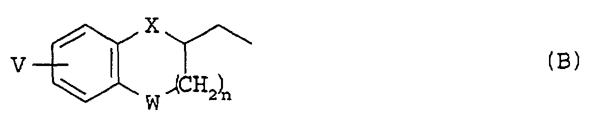 dans lesquelles
V représente un atome d'hydrogène ou d'halogène, un groupe (C₁-C₃)alkyle ou un ou deux groupes (C₁-C₃)alcoxy,
W et X représentent chacun, respectivement, soit deux atomes d'oxygène, soit un atome d'oxygène et un groupe CH₂, soit un groupe CH₂ et un atome d'oxygène, soit un atome d'oxygène et un groupe CO,
n représente le nombre 0 ou 1,
R représente soit un groupe propyle lorsque U représente un groupe 2,3-dihydro-1*H*-indén-2-yle de formule générale (A), soit un atome d'hydrogène ou un groupe (C₁-C₃)alkyle lorsque
U représente un groupe hétérocyclique de formule générale (B),
Y représente un ou plusieurs atomes ou groupes choisis parmi les suivants : hydrogène, halogène, (C₁-C₃)alkyle et (C₁-C₃)alcoxy, et
Z représente deux atomes d'hydrogène ou un atome d'oxygène.

2. Composé selon la revendication 1, **caractérisé en ce qu'**il répond à la formule générale (IA) 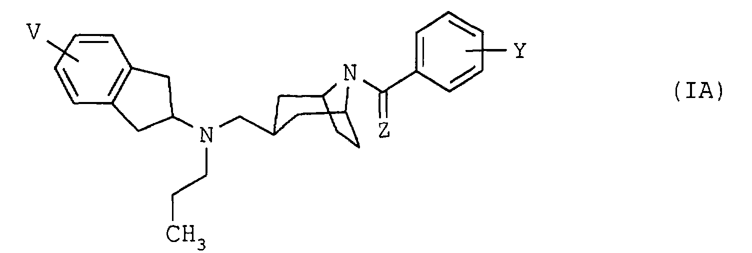 dans laquelle V, Y et Z sont tels que définis dans la revendication 1.

3. Composé selon la revendication 1, **caractérisé en ce qu'**il répond à la formule générale (IB) 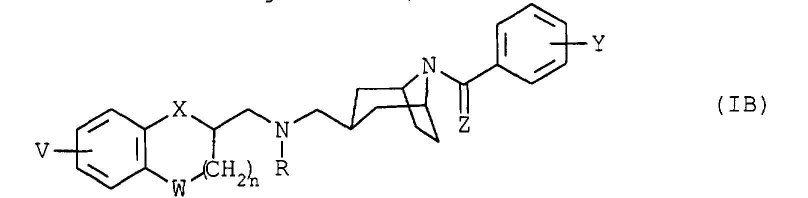 dans laquelle V, W, X, Y et Z sont tels que définis dans la revendication 1.

4. Composé selon la revendication 3, **caractérisé en ce que** V représente un atome de chlore en position 7, W représente un atome d'oxygène, W représente un atome d'oxygène, X représente un atome d'oxygène, Y représente un atome d'hydrogène, Z représente un atome d'oxygène, n représente le nombre 1, et R représente un atome d'hydrogène.

5. Médicament **caractérisé en ce qu'**il consiste en un composé selon l'une des revendications 1 à 3.

6. Composition pharmaceutique **caractérisée en ce qu'**elle contient un composé selon l'une des revendications 1 à 3, associé à un excipient.

## Claims

1. Compound, in the form of a pure geometrical isomer or a mixture of such isomers, corresponding to the general formula (I) 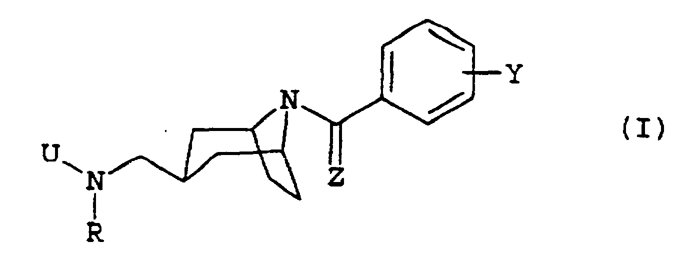 in which
U represents
A) either a 2,3-dihydro-1*H*-inden-2-yl group of general formula (A) 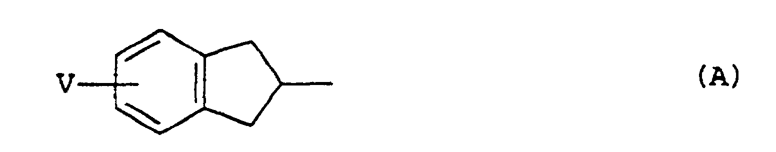
B) or a heterocyclic group of general formula (B) 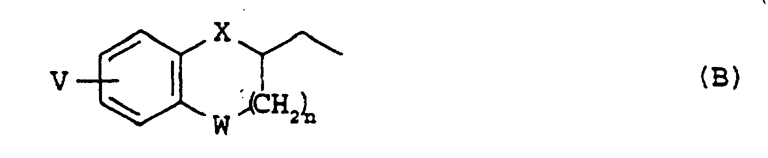 in which
V represents a hydrogen or halogen atom, a (C₁-C₃)alkyl group or one or two (C₁-C₃)alkoxy groups,
W and X each represent, respectively, either two oxygen atoms, or an oxygen atom and a CH₂ group, or a CH₂ group and an oxygen atom, or an oxygen atom and a CO group,
n represents the number 0 or 1,
R represents either a propyl group when U represents a 2,3-dihydro-1*H*-inden-2-yl group of general formula (A), or a hydrogen atom or a (C₁-C₃)alkyl group when U represents a heterocyclic group of general formula (B),
Y represents one or more atoms or groups chosen from the following: hydrogen, halogen, (C₁-C₃)alkyl and (C₁-C₃)alkoxy, and
Z represents two hydrogen atoms or an oxygen atom.

2. Compound according to Claim 1, **characterized in that** it corresponds to the general formula (IA) 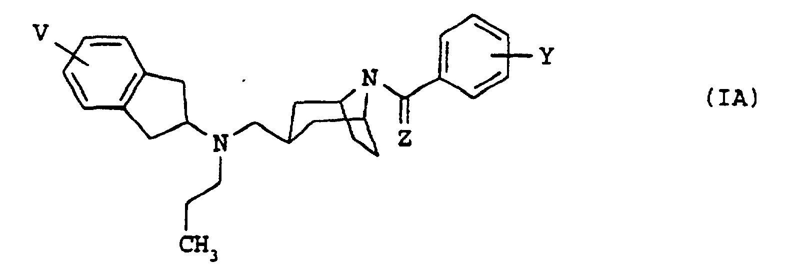 in which V, Y and Z are as defined in Claim 1.

3. Compound according to Claim 1, **characterized in that** it corresponds to the general formula (IB) 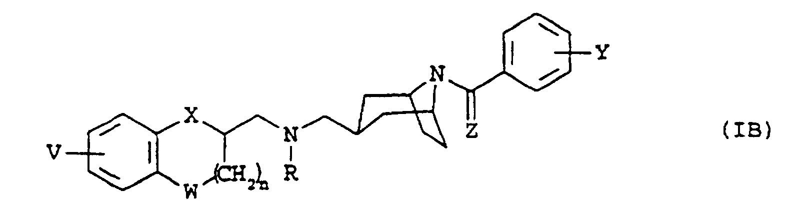 in which V, W, X, Y and Z are as defined in Claim 1.

4. Compound according to Claim 3, **characterized in that** V represents a chlorine atom in position 7, W represents an oxygen atom, X represents an oxygen atom, Y represents a hydrogen atom, Z represents an oxygen atom, n represents the number 1, and R represents a hydrogen atom.

5. Medicament, **characterized in that** it consists of a compound according to one of Claims 1 to 3.

6. Pharmaceutical composition, **characterized in that** it contains a compound according to one of Claims 1 to 3, combined with an excipient.

## Patentansprüche

1. Verbindung der allgemeinen Formel (I) in Form des reinen geometrischen Isomeren oder einer Mischung solcher Isomeren 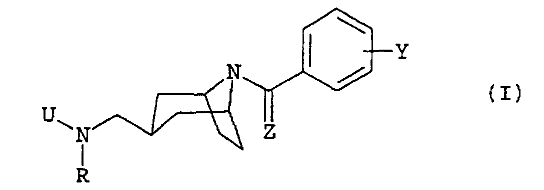 in der
U
A) entweder eine 2,3-Dihydro-lH-inden-2-yl-gruppe der allgemeinen Formel (A) 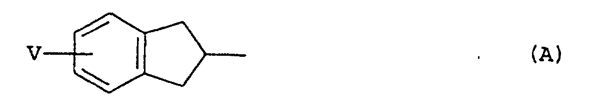
B) oder eine heterocyclische Gruppe der allgemeinen Formel (B) 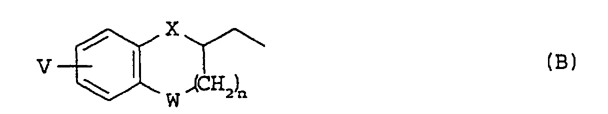 darstellt, worin
V ein Wasserstoffatom oder ein Halogenatom, eine (C₁-C₃)-Alkylgruppe oder eine oder zwei (C₁-C₃)-Alkoxygruppen,
W und X jeweils entweder zwei Sauerstoffatome oder ein Sauerstoffatom und eine CH₂-Gruppe oder eine CH₂-Gruppe und ein Sauerstoffatom oder ein Sauerstoffatom und eine CO-Gruppe,
n die Zahl 0 oder 1 bedeuten,
R entweder eine Propylgruppe, wenn U eine 2,3-Dihydro-1*H*-inden-2-yl-gruppe der allgemeinen Formel (A) darstellt, oder ein Wasserstoffatom oder eine (C₁-C₃)-Alkylgruppe, wenn U eine heterocyclische Gruppe der allgemeinen Formel (B) darstellt, bedeutet,
Y ein oder mehrere Atome oder Gruppen ausgewählt aus: Wasserstoff, Halogen, (C₁-C₃)-Alkyl und (C₁-C₃)-Alkoxy bedeutet, und
Z zwei Wasserstoffatome oder ein Sauerstoffatom darstellt.

2. Verbindung nach Anspruch 1, **dadurch gekennzeichnet, daß** sie der allgemeinen Formel (IA) entspricht 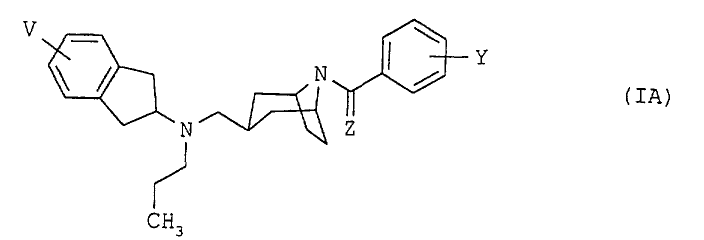 in der V, Y und Z die in Anspruch 1 angegebenen Bedeutungen besitzen.

3. Verbindung nach Anspruch 1, **dadurch gekennzeichnet, daß** sie der allgemeinen Formel (IB) entspricht 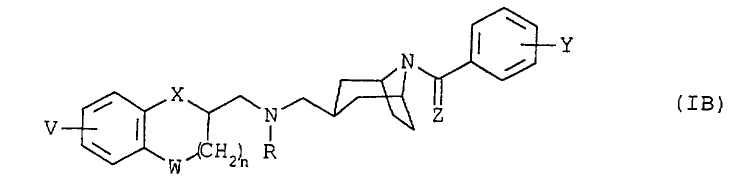 in der V, W, X, Y und Z die in Anspruch 1 angegebenen Bedeutungen besitzen.

4. Verbindung nach Anspruch 3, **dadurch gekennzeichnet, daß** V ein Chloratom in der 7-Stellung, W ein Sauerstoffatom, W ein Sauerstoffatom, X ein Sauerstoffatom, Y ein Wasserstoffatom, Z ein Sauerstoffatom, n die Zahl 1 und R ein Wasserstoffatom bedeuten.

5. Arzneimittel, **dadurch gekennzeichnet, daß** es aus einer Verbindung nach einem der Ansprüche 1 bis 3 besteht.

6. Pharmazeutische Zubereitung, **dadurch gekennzeichnet, daß** sie eine Verbindung nach einem der Ansprüche 1 bis 3 zusammen mit einem Trägermaterial enthält.
